# EUROPEAN PATENT APPLICATION

(11) **EP 4 484 419 A1**
(43) Date of publication of application: **01.01.2025**
(21) Application number: 23760353.5
(22) Date of filing: 21.02.2023
(51) Int. Cl.: C07D 405/04, C07D 409/04, C07D 493/04, C07D 495/04, C07D 409/14, C07D 403/04, C07D 471/04, C07D 491/048, C07D 405/14, H10K 85/60

(54) **ORGANIC COMPOUND AND ORGANIC ELECTROLUMINESCENT DEVICE USING SAME**

(30) Priority: 22.02.2022 KR 20220023188
(71) Applicant: Solus Advanced Materials Co., Ltd., Iksan-si, Jeollabuk-do 54584 (KR)
(72) Inventor: BAE, Hyungchan, Yongin-si, Gyeonggi-do 16858 (KR); SIM, Jaeyi, Yongin-si, Gyeonggi-do 16858 (KR); CHO, Areum, Yongin-si, Gyeonggi-do 16858 (KR)
(74) Representative: Office Freylinger
(86) International application number: PCT/KR2023/002442
(87) International publication number: WO 2023/163479

(57) **Abstract**

The present invention relates to a novel compound with excellent carrier transport ability, light-emitting ability, and thermal stability, and an organic electroluminescent device that includes the compound in at least one organic material layer and thus has improved characteristics such as improved luminous efficiency, driving voltage, and lifespan.

## Description

### TECHNICAL FIELD

The present invention relates to a novel organic light-emitting compound and an organic electroluminescent element using the same and more particularly, to: a compound having excellent electron transport ability, light emitting ability, and thermal stability, and an organic electroluminescent element including the compound in one or more organic layers and thus improved in terms of characteristics such as a low voltage, a high efficiency, and a long lifespan.

### BACKGROUND ART

Starting from Bernanose's observation of light emission from organic thin films in the 1950s, the study of organic electroluminescent ("EL") elements led to blue electroluminescence using anthracene monocrystals in 1965, and Tang suggested in 1987 an organic EL element in a stack structure which may be divided into functional layers of hole layers and light emitting layers. Then, in order to develop high efficiency, long lifespan organic EL elements, organic layers each having distinctive characteristics have been introduced in the elements, leading to the development of specialized materials used therein.

In organic EL elements, upon application of voltage between two electrodes, holes are injected from an anode (e.g., positive electrode) to an organic layer and electrons are injected from a cathode (e.g., negative electrode) into the organic layer. Injected holes and electrons meet each other to form excitons, and light emission occurs when the excitons fall to a ground state. In such a case, materials used for the organic layer may be classified into, for example, luminescent (e.g., light emitting) materials, hole injection materials, hole transport materials, electron transport materials and electron injection materials depending on their function.

Light emitting layer materials of an organic EL element may be classified into, for example, blue-, green- and red-light emitting materials depending on their emission colors. Besides, yellow and orange light emitting materials may also be used as such a light emitting material for realizing better natural colors. In addition, a host/dopant system may be employed in the light emitting material to increase color purity and luminescence efficiency through energy transferring. Dopant materials may be classified into fluorescent dopants using organic materials and phosphorescent dopants using metal complex compounds which include heavy atoms such as Ir and Pt. The developed phosphorescent materials may improve the luminescence efficiency theoretically up to four times as compared to fluorescent materials, so attention is given to phosphorescent dopants as well as phosphorescent host materials. To date, NPB, BCP and Alq₃, for example, are widely known as materials used in the hole injection layer, the hole transporting layer, the hole blocking layer and the electron transporting layer, and anthracene derivatives have been reported as fluorescent dopant/host materials for light emitting materials. Particularly, metal complex compounds including Ir, such as FIrpic, Ir(ppy)₃, and Ir(btp)₂(acac), are known as phosphorescent dopant materials for efficiency improvement among light emitting materials, and they are used as blue, green and red dopant materials. Up to this day, CBP has shown excellent properties as a phosphorescent host material.

However, conventional materials, despite their good luminescence properties, have low glass transition temperatures and poor thermal stability and thus are not satisfactory in terms of lifespan characteristics of organic EL elements. Accordingly, there is a demand for the development of an organic layer material having excellent performance.

### DETAILED DESCRIPTION OF THE INVENTION

### TECHNICAL OBJECTIVES

The present invention is directed to a novel organic compound applicable to organic electroluminescent elements and having excellent characteristics such as hole injection and transport ability, electron injection and transport ability, and luminescence ability.

The present invention is also directed to an organic electroluminescent element including the novel organic compound described above and thereby has a low driving voltage, a high luminescence efficiency, and an improved lifespan.

Other objectives and advantages of the present invention may be more clearly described by the following detailed description and claims.

### TECHNICAL MEANS TO SOLVE THE PROBLEM

To achieve the above objectives, the present invention provides a compound represented by the following Chemical Formula 1: 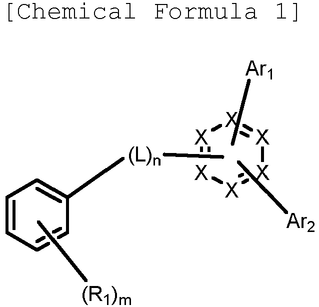 wherein in Chemical Formula 1,
a plurality of X are the same as or different from each other, and are each independently CR₂ or N, provided that at least two of the plurality of X are N,
R₂ is selected from the group consisting of hydrogen, deuterium (D), halogen, a cyano group, a nitro group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ monoarylphosphinyl group, a C₆ to C₆₀ diarylphosphinyl group, a C₆ to C₆₀ arylamine group, a C₅ to C₆₀ arylheteroarylamine group, and a heteroarylamine group having 5 to 60 nuclear atoms,
L is a single bond, or is selected from the group consisting of a C₆-C₁₈ arylene group and a heteroarylene group having 5 to 18 nuclear atoms,
R₁ is selected from the group consisting of a C₆ to C₆₀ aryl group, and a heteroaryl group having 5 to 60 nuclear atoms,
m is an integer of 1 or more, n is an integer of 0 to 3,
Ar₂ is selected from the group consisting of a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ monoarylphosphinyl group, a C₆ to C₆₀ diarylphosphinyl group, a C₆ to C₆₀ arylamine group, a C₅ to C₆₀ arylheteroarylamine group, and a heteroarylamine group having 5 to 60 nuclear atoms,
Ar₁ is substituent represented by the following Chemical Formula 2, 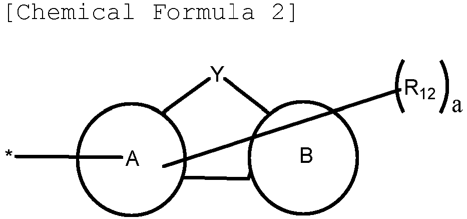 in Chemical Formula 2,
   * indicates a site where a bond with Chemical Formula 1 is made,
   Y is O, S or NR₁₁,
   Ring A and Ring B are the same as or different from each other, each independently being a C₅ to C₁₈ monocyclic or polycyclic hydrocarbon ring that may or may not include heteroatoms,
   R₁₁ and R₁₂ are the same as or different from each other, each independently being selected from: a C₁ to C₁₂ alkyl group, a C₆ to C₁₂ aryl group, and a heteroaryl group having 5 to 12 nuclear atoms, or combine with an adjacent group to form a fused ring,
   a is an integer of 0 to 2,
   the arylene and heteroarylene groups of L; the aryl, heteroaryl groups of R₁; the alkyl, alkenyl, alkynyl, cycloalkyl, heterocycloalkyl, aryl, heteroaryl, alkyloxy, aryloxy, alkylsilyl, arylsilyl, alkylboron, arylboron, arylphosphine, monoarylphosphinyl, diarylphosphinyl, arylamine, arylheteroarylamine, and heteroarylamine groups of R₂, R₁₁ to R₁₂ and Ar₂ are optionally each independently unsubstituted or substituted with at least one selected from the group consisting of a deuterium (D), a halogen, a cyano group, a nitro group, a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 nuclear atoms, a C₆-C₆₀ aryl group, a heteroaryl group having 5 to 60 nuclear atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a C₁-C₄₀ arylphosphine group, a C₆-C₆₀ aryl phosphine oxide group, a C₆-C₆₀ arylamine group, a C₅-C₆₀ 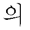 arylheteroarylamine group, and a heteroarylamine group having 5 to 60 nuclear atoms, and provided that when the substituent is present in a plural number, they are optionally the same or different from each other, and
   provided that the total number of carbon atoms contained in Ar₁ substituted or unsubstituted with the substituents comprises 16 to 24.

In addition, the present invention also provides an organic electroluminescent element including: an anode, a cathode, and one or more organic layers disposed between the anode and the cathode, wherein at least one of the one or more organic layers includes the compound represented by Chemical Formula 1.

In such a case, the organic layer including the compound represented by Chemical Formula 1 may be selected from a light emitting layer, a light emitting auxiliary layer, a hole injection layer, a hole transport layer, an electron injection layer, a lifespan improvement layer, an electron transport layer, and an auxiliary electron transport layer. In such a case, the compound represented by Chemical Formula 1 may be included as a material for at least one of a phosphorescent host material of a light emitting layer, an electron transport layer and an auxiliary electron transport layer.

### EFFECTS OF THE INVENTION

According to an embodiment of the present invention, a compound represented by Chemical Formula 1 has excellent characteristics such as carrier transport ability, luminescence ability, heat resistance, and the like, and thereby is applicable as an organic layer material of an organic electroluminescent element.

In particular, when the compound represented by Chemical Formula 1 of the present invention is used as a phosphorescent host, an electron transport layer material or an auxiliary electron transport layer material, it may exhibit high thermal stability, low driving voltage, rapid mobility, high current efficiency and long lifespan characteristics compared to conventional host materials or electron transport materials.

Accordingly, the organic electroluminescent element including the compound of Chemical Formula 1 may be significantly improved in aspects such as excellent light emitting performance, low driving voltage, long lifespan, and high efficiency, and thus may be effectively applied to full color display panels and the like.

Effects according to the present invention are not limited by the description exemplified above, and more diverse effects are included in the present specification.

### DETAILED DESCRIPTION OF THE INVENTION

Hereinafter, the present invention will be described in detail.

### <Novel organic compound>

The present invention provides a novel aryl-based compound having excellent thermal stability, carrier transport ability, and luminescence ability, and aims to achieve simultaneously low voltage, high efficiency, and long lifespan of a device including the compound.

According to the present invention, the novel organic compound represented by Chemical Formula 1 has a basic skeleton structure in which a plurality of aryl groups (e.g., R₁-containing ring), and a ring compound having a non-covalent electron pair (e.g., a lone pair) such as a dibenzo-/carbazole-based moiety, are bonded to an electron withdrawing group (EWG) having excellent electron transport ability.

The compound of chemical formula 1 having the structure described above structurally increases the electron density of EWG having electron transport ability by centering on an azine group (e.g., triazine, pyrimidine etc), which is a type of EWG group, and substituting a ring compound having a shared electron pair on one side of the azine group and substituting a plurality of aryl groups on the other side. Accordingly, the electron transport ability is further improved compared to existing known material structures, resulting in an effect of increased efficiency. Further, the compound has a rigid chemical structure due to the dibenzo-based or carbazole-based ring compound bonded to the EWG group, and thus is excellent in terms of high glass transition temperature (Tg) and thermal stability. And in the present invention, through the chemical design in which the conjugation length is relatively increased by the dibenzo-based/carbazole-based ring compound bonded to the EWG, the stability of the molecule can be increased, thereby providing a higher lifespan of the device than the existing known technology. Furthermore, the compound of the present invention can have physicochemical properties more suitable for electron injection and electron transport by introducing an azine group having a strong electron-withdrawing ability (EWG) to improve the electron mobility rate.

Moreover, since the compound represented by Chemical Formula 1 of the present invention has a high triplet energy, it is possible to prevent excitons generated in the light-emitting layer from diffusing (migrating) into an adjacent electron transport layer or hole transport layer. Accordingly, the number of excitons contributing to light emission in the light-emitting layer may be increased, thus improving luminous efficiency of the device, and durability and stability of the device may be improved, such that the lifespan of the device may be effective increased. Most of the developed materials allow low-voltage operation, thereby exhibiting physical characteristics with improved lifespan.

As described above, the compound represented by Chemical Formula 1 of the present invention may be applicable as an organic layer material of an organic electroluminescent device, preferably a light-emitting layer material (a blue, a green and/or a red phosphorescent host material), an electron transport/injection layer material, a hole transport/injection layer material, a light-emitting auxiliary layer material, an auxiliary electron transport layer, and/or a lifespan enhancement layer material. In addition, the organic electroluminescent device including the compound of the above chemical formula may be greatly improved in terms of the performance and lifespan characteristics, and the performance a full color organic electroluminescent panel to which the organic electroluminescent device is applied may also be maximized.

According to the present invention, the compound represented by Chemical Formula 1 has a basic skeleton structure in which a dibenzo-/carbazole-based ring compound (e.g., Ar₁) and a plurality of aryl groups (e.g., R₁-containing ring) are bonded to an electron withdrawing group (EWG) having excellent electron transport ability, such as a nitrogen-containing heterocycle as a center.

In Chemical Formula 1, the nitrogen-containing heterocycle (e.g., a ring containing X) may be a monocyclic heteroaryl group (e.g., azine) containing at least two nitrogen atoms. For an example of the nitrogen-containing heteroaromatic ring (e.g., the ring containing X), a plurality of X may be the same as or different from each other, and may each independently be N or CR₂, provided that at least two of the plurality of X are N. For a specific example, a plurality of X includes two to three nitrogen atoms (N), preferably three nitrogen atoms (N). As such, since the heterocycle containing two to three nitrogen atoms (N) is included, more excellent electron absorption characteristics may be exhibited, which is advantageous for electron injection and transport.

In an embodiment of the present invention, the nitrogen-containing heterocycle (e.g., X-containing ring) may be embodied into any one of the following structural formulas. However, it is not particularly limited thereto. 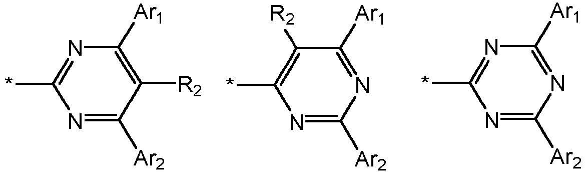 wherein in the above structural formulas,
* indicates a site where a bond with Chemical Formula 1 is made.

Herein, CR₂ in the nitrogen-containing heteroaromatic ring (e.g., X-containing ring) may be plural in number, and in such a case, the plurality of R₂ may be the same as or different from each other and may each independently be selected from: hydrogen, deuterium (D), halogen, a cyano group, a nitro group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₃ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ monoarylphosphinyl group, a C₆ to C₆₀ diarylphosphinyl group, a C₆ to C₆₀ arylamine group, a C₅ to C₆₀ arylheteroarylamine group, and a heteroarylamine group having 5 to 60 nuclear atoms. Specifically, R₂ may preferably be selected from: hydrogen, deuterium, a cyano group, a C₁ to C₄₀ alkyl group, a C₆ to C₆₀ aryl group, and a heteroaryl group having 5 to 60 nuclear atoms.

The nitrogen-containing heterocycle (e.g., X-containing ring) according to the present invention may each be substituted with various substituents, e.g., Ar₄ to Ar₂. Such Ar₂ and Ar₂ may the same as or different from each other, any one of Ar₂ and Ar₂ may have a dibenzo-based ring or carbazole based ring.

In an embodiment of the present invention, Ar₁ may be a substituent represented by the following Chemical Formula 2. 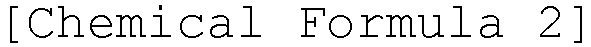 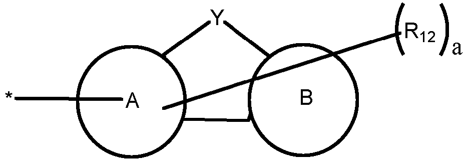 in Chemical Formula 2,
* may mean a site in which a bond with Chemical Formula 1 is made,
Y is O, S or NR₁₁,
Ring A and Ring B may be the same as or different from each other, each independently a C₅ to C₁₈ monocyclic or polycyclic hydrocarbon ring that may or may not include heteroatoms. The ring A and ring B may be condensed or fused monocyclic or polycyclic hydrocarbon rings or nitrogen-containing rings known in the art, and may be, for example, monocyclic or polycyclic alicyclic rings, monocyclic or polycyclic heteroalicyclic rings, monocyclic or polycyclic aromatic rings, or monocyclic or polycyclic heteroaromatic rings. Preferably, they may be a monocyclic or polycyclic aromatic ring having 6 to 12 carbon atoms, or a monocyclic or polycyclic heteroaromatic ring having 5 to 12 nuclear atoms containing at least one heteroatom. Here, the heteroatom may be selected from the group consisting of N, O, and S.
R₁₁ and R₁₂ are the same as or different from each other, each independently being selected from: a C₁ to C₁₂ alkyl group, a C₆ to C₁₂ aryl group, and a heteroaryl group having 5 to 12 nuclear atoms, or combine with an adjacent group to form a fused ring,
a is an integer of 0 to 2. when a is 0, R₁₂ means hydrogen, and when a is 1 to 2, R₁₂ may have the aforementioned substituents. When a plurality of R₁₂ is present, they may be the same as or different from each other.

In the present invention, provided that Ar₁ represented by the Chemical Formula 2 may be substituted or unsubstituted with a substituent described below, and the total number of carbon atoms contained in the substituted and/or unsubstituted Ar₁ includes 16 to 24.

In an embodiment of the present invention, Ar₁ may be more specifically embodied as any one selected from the following structural formulas. However, it is not limited thereto.

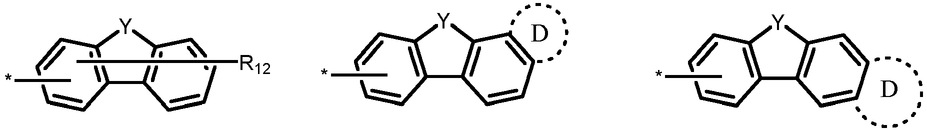

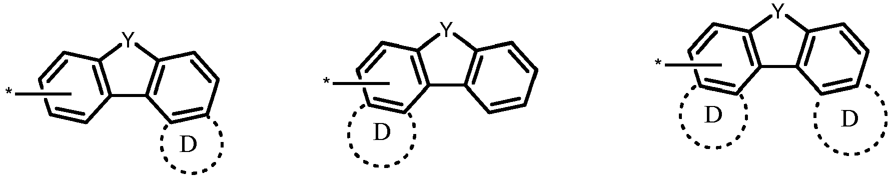

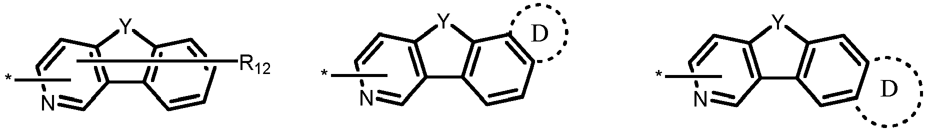

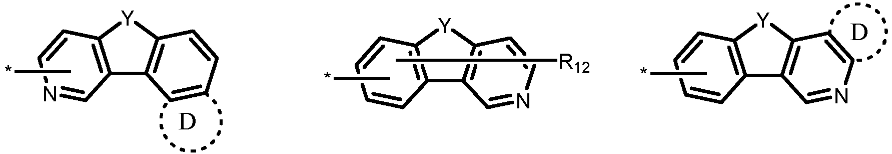

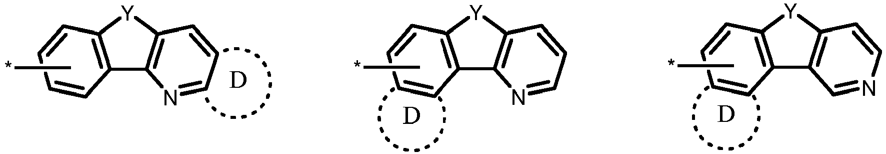

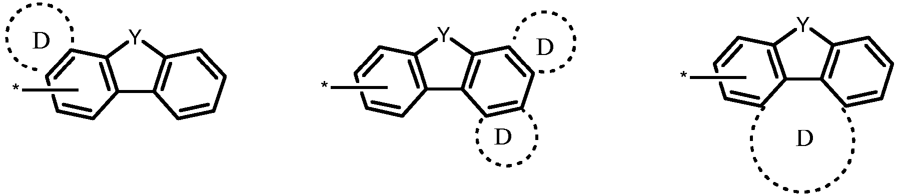

In the above structural formulas,
* may indicate a site where a bond to Chemical Formula 1 is made,
Ring D may be the same as or different from each other, each independently a monocyclic or polycyclic hydrocarbon ring group containing or not containing heteroatoms, and
Y and R₁₂ are each as defined in Chemical Formula 1.

For one specific example, Ar₁ may be any one selected from the following structural formulas.

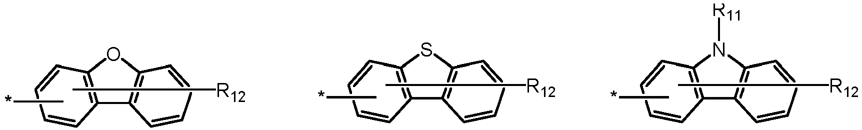

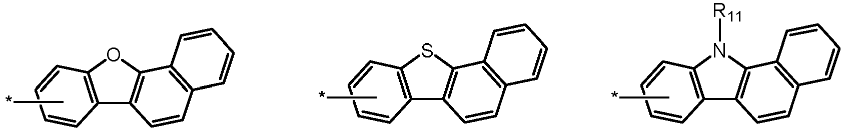

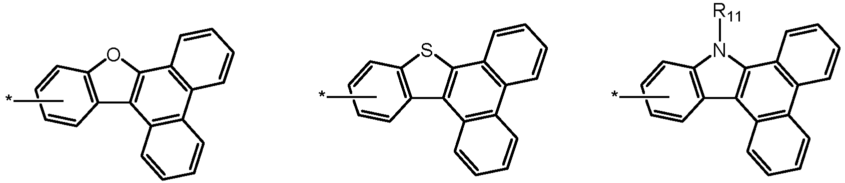

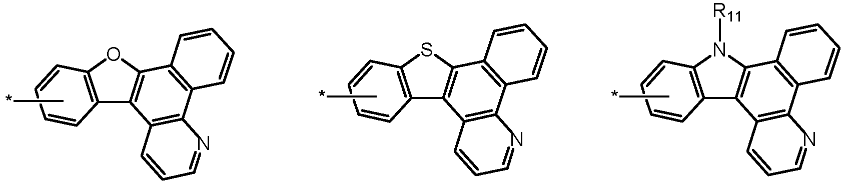

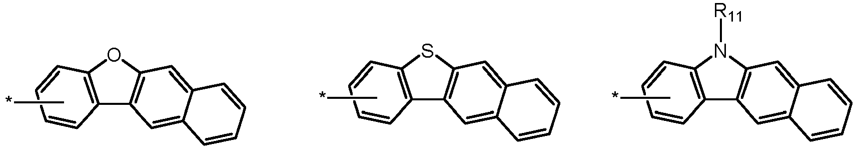

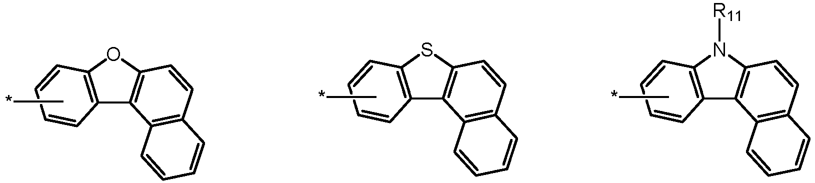

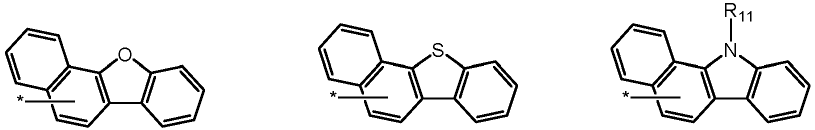

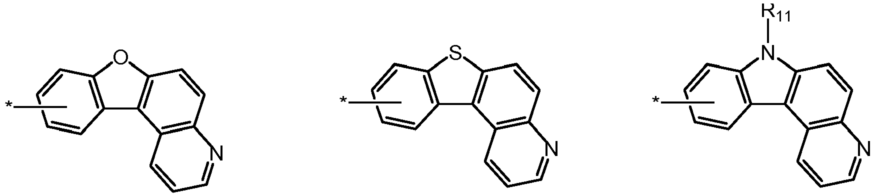

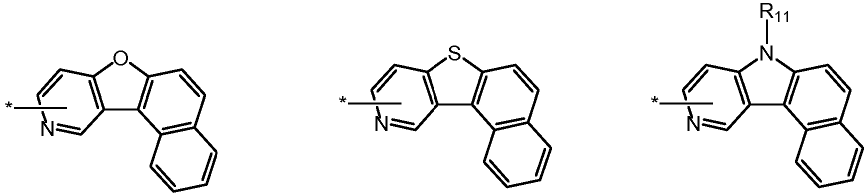

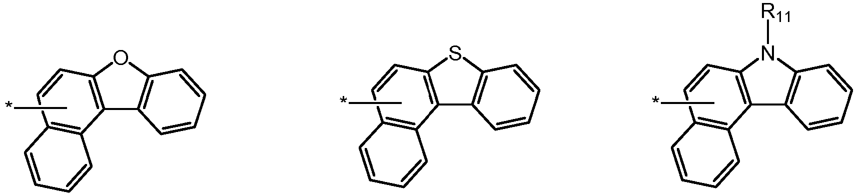

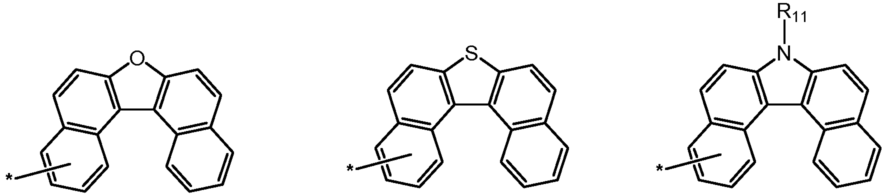

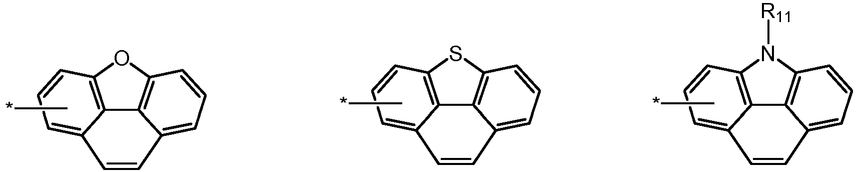

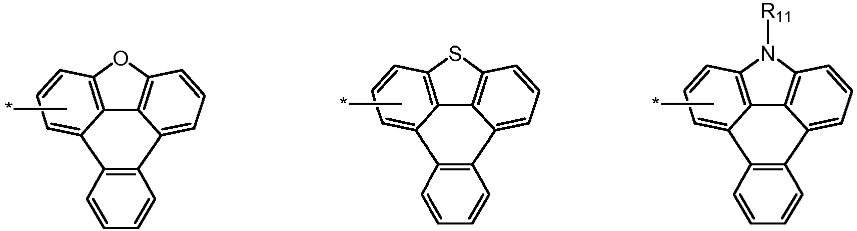

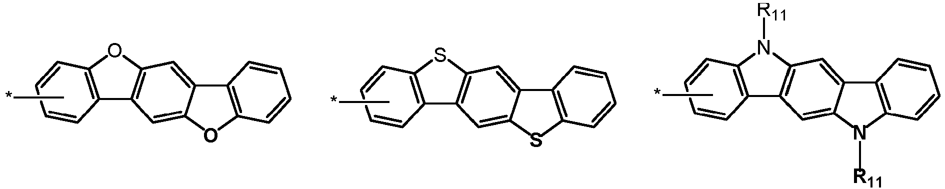

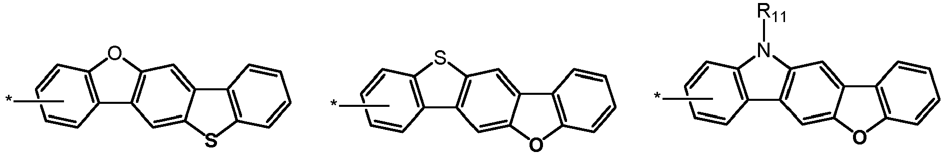

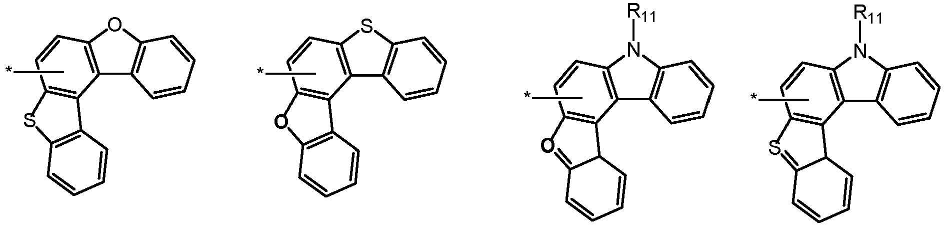

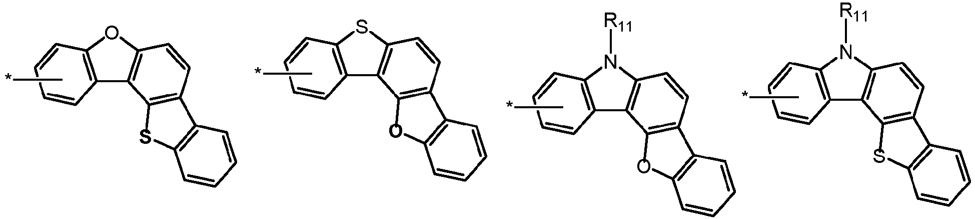

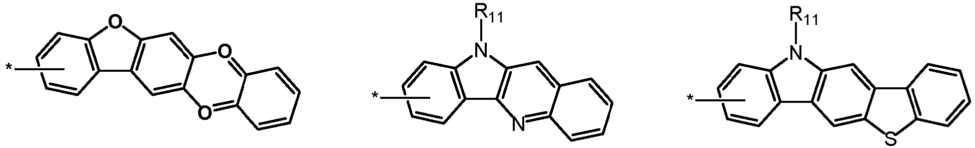

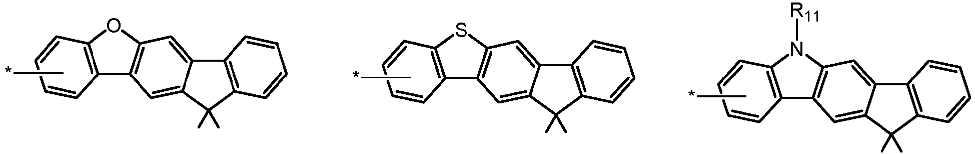

In the above structural formulas,
* may indicate a site where a bond to Chemical Formula 1 is made, and R₁₁ and R₁₂ are each as defined in Chemical Formula 2.

In addition, although not shown in the above structural formulas, at least one substituent known in the art (e.g., the same as the definition of R₂) may be substituted.

Ar₂ in the nitrogen-containing heterocycle (e.g., X-containing ring) may be selected from: a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₃ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ monoarylphosphinyl group, a C₆ to C₆₀ diarylphosphinyl group, a C₆ to C₆₀ arylamine group, a C₅ to C₆₀ arylheteroarylamine group, and a heteroarylamine group having 5 to 60 nuclear atoms. Specifically, Ar₂ may be selected from: a C₆ to C₆₀ aryl group, and a heteroaryl group having 5 to 60 nuclear atoms, more specifically, Ar₂ may preferably be selected from: a C₆ to C₁₈ aryl group, and a heteroaryl group having 5 to 18 nuclear atoms.

For one specific example, Ar₂ may be any one selected from the following structural formulas.

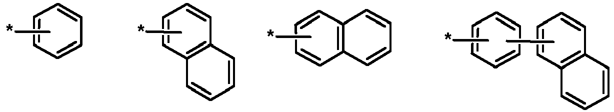

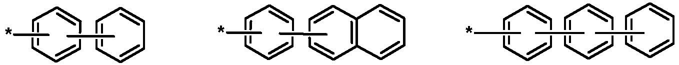

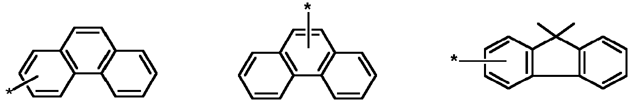

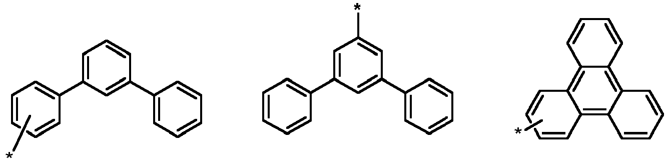

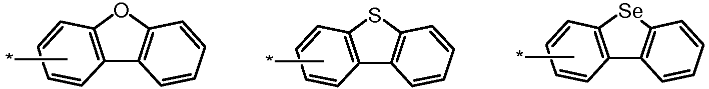

In the above structural formulas,
* may indicate a site where a bond to Chemical Formula 1 is made.

The nitrogen-containing heterocycle according to the present invention may be bonded to a plurality of aryl groups (R₁), which are connected via a direct bond (n=0) or a separate linker (L). As such, when a separate linker (L) is present between the nitrogen-containing heteroaromatic ring and the plurality of the aryl groups, a HOMO region may be expanded to give a benefit to a HOMO-LUMO distribution, and charge transfer efficiency may be increased through an appropriate overlap of HOMO-LUMO.

The linker (e.g., L) are not particularly limited, and may be a common divalent group linker known in the art. Specifically, L may be a single bond (e.g., direct bond) or selected from: a C₆ to C₁₈ arylene group and a heteroarylene group having 5 to 18 nuclear atoms. More specifically, L may be selected from: a C₆ to C₁₂ arylene group and a heteroarylene group having 5 to 12 nuclear atoms.

n is an integer of 0 to 3. when n is 0, L means a single bond, and when a is 1 to 2, a plurality of Ls may be the same or different from each other.

Specific examples of the arylene group and the heteroarylene group may include, for example, a phenylene group, a biphenylene group, a naphthylene group, an anthracenylene group, an indenylene group, a pyrantrenylene group, a carbazolylene group, a thiophenylene group, an indolylene group, a purinylene group, a quinolinylene group, a pyrrolylene group, an imidazolylene group, an oxazolylene group, a thiazolylene group, a triazolylene group, a pyridinylene group, and a pyrimidinylene group. And specific examples of the heteroarylene group may include, for examples, a dibenzofuran-based moiety, a dibenzothiophene-based moiety, and/or a dibenzoselenophenebased moiety.

The compound of the Chemical Formula 1 according to the present invention includes a plurality of aryl groups/heteroaryl groups (e.g., R₁-containing rings) bonded to one side of a nitrogen-containing heterocycle (e.g., X containing ring).

Specifically, R₁ may be selected from the group consisting of a C₆ to C₆₀ aryl group and a heteroaryl group having a nuclear atom number of 5 to 60. The number (e.g., m) of aryl groups/heteroaryl groups may be an integer of 1 or more, and may be preferably 1 to 5. Here, when m is 2 to 5, a plurality of R1s may be the same or different.

In an embodiment of the present invention, R₁ is a C₆ to C₁₂ aryl group, m is an integer of 1 to 5, and the total number of carbon atoms contained in R₁ substituted or unsubstituted with a substituent described below includes at least 18.

In the above Chemical Formula 1, the arylene and heteroarylene groups of L; the aryl, heteroaryl groups of R₁; the alkyl, alkenyl, alkynyl, cycloalkyl, heterocycloalkyl, aryl, heteroaryl, alkyloxy, aryloxy, alkylsilyl, arylsilyl, alkylboron, arylboron, arylphosphine, monoarylphosphinyl, diarylphosphinyl, arylamine, arylheteroarylamine, and heteroarylamine groups of R₂, R₁₁ to R₁₂ and Ar₂ are optionally each independently unsubstituted or substituted with at least one selected from the group consisting of a deuterium (D), a halogen, a cyano group, a nitro group, a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 nuclear atoms, a C₆-C₆₀ aryl group, a heteroaryl group having 5 to 60 nuclear atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a C₁-C₄₀ arylphosphine group, a C₆-C₆₀ aryl phosphine oxide group, a C₆-C₆₀ arylamine group, a C₅-C₆₀ 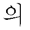 arylheteroarylamine group, and a heteroarylamine group having 5 to 60 nuclear atoms, and provided that when the substituent is present in a plural number, they are optionally the same or different from each other,

In an embodiment of the present invention, the compound represented by Chemical Formula 1 may be represented by any one of the following Chemical Formulas 3 to 9 according to the type of the linker (L) and the cyclic compound having a lone pair of electrons, specifically a dibenzo-based/carbazole-based moieties (e.g., Ar₁). However, it is not limited thereto.

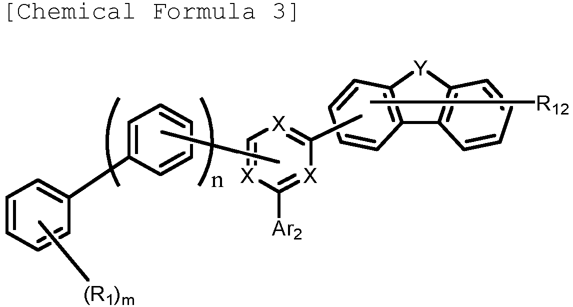

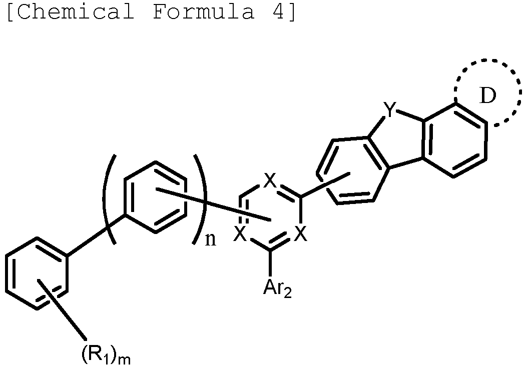

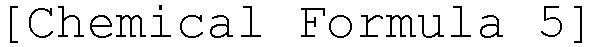

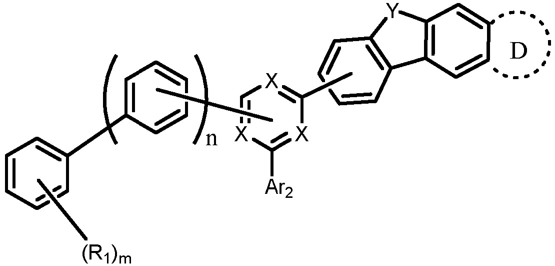

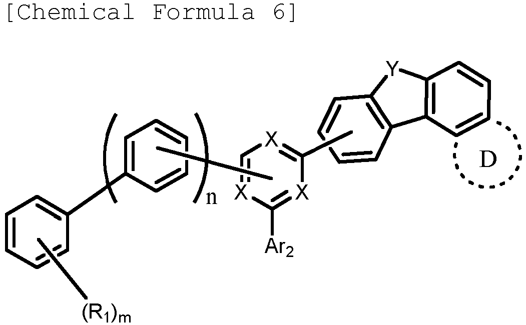

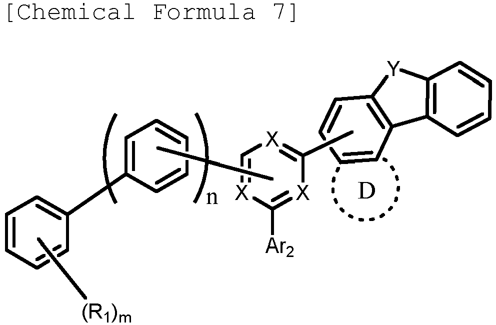

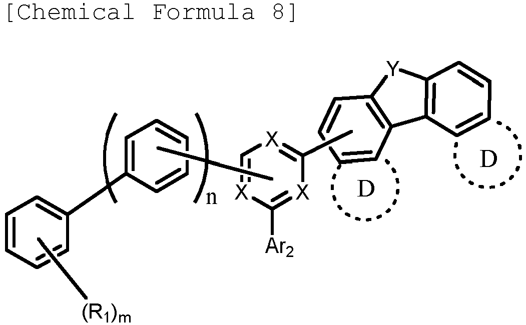

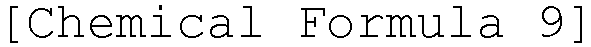

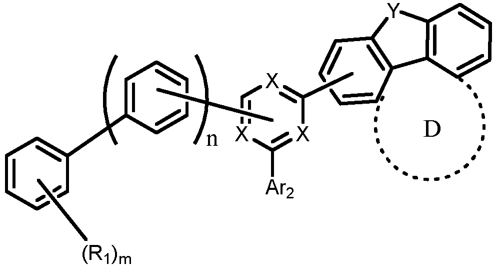

In above Chemical Formulas,
X, Y, Ar₂, R₁, n and m are each as defined in Chemical Formula 1, and
Ring D is a monocyclic or polycyclic hydrocarbon ring group that may or may not include heteroatoms.

In another embodiment of the present invention, the compound represented by Chemical Formula 1 may be represented by any one of the following Chemical Formulas 10 to 21 according to the number of aryl groups (R₁) introduced into the molecule and their bonding positions. However, it is not limited thereto.

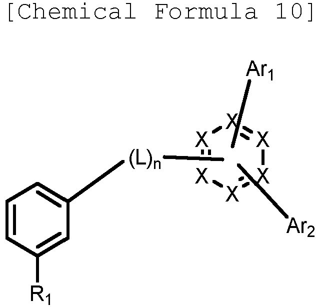

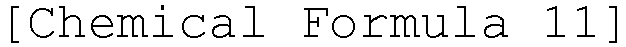

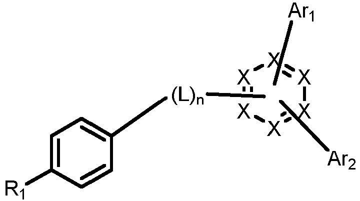

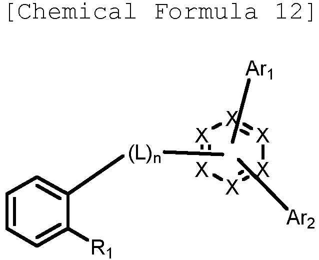

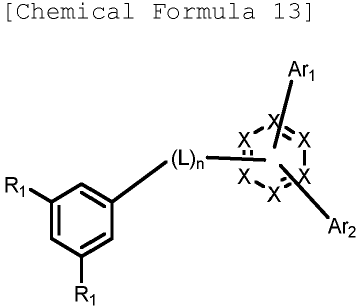

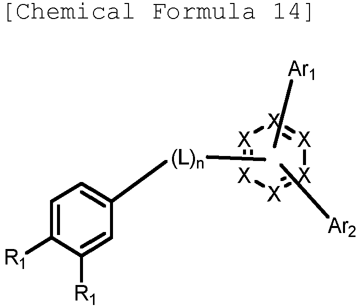

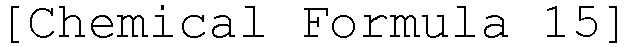

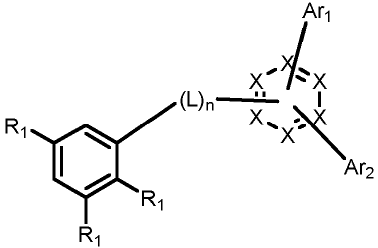

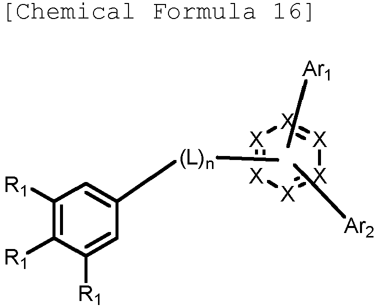

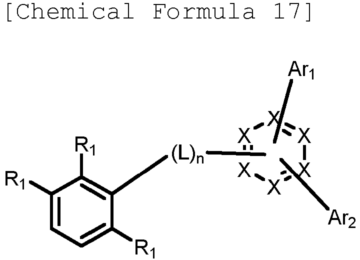

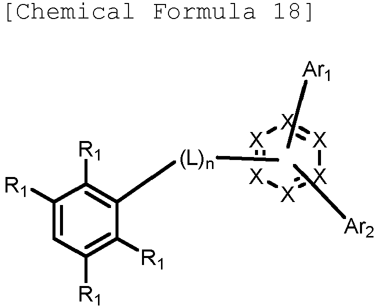

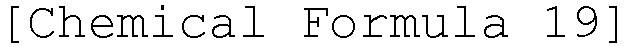

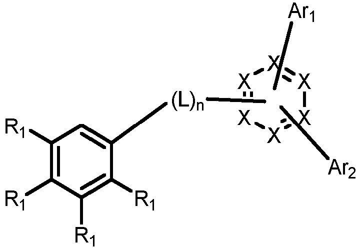

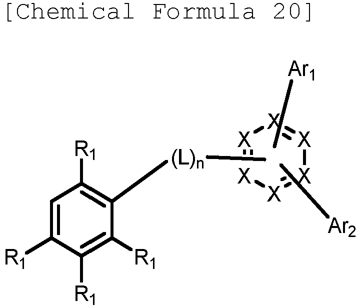

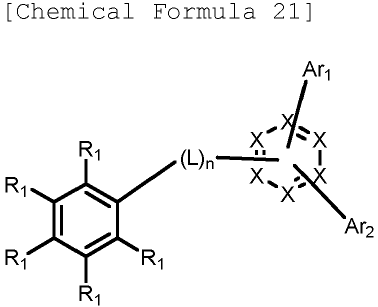

In above Chemical Formulas,
X, Ar₂, Ar₂, L, R₁, and n are each as defined in Chemical Formula 1.

The compound represented by Chemical Formula 1 of the present invention described above may be further embodied as compounds represented by the following compounds represented by, for example, 1 to 93. However, the compound represented by Chemical Formula 1 of the present invention is not limited to those exemplified below.

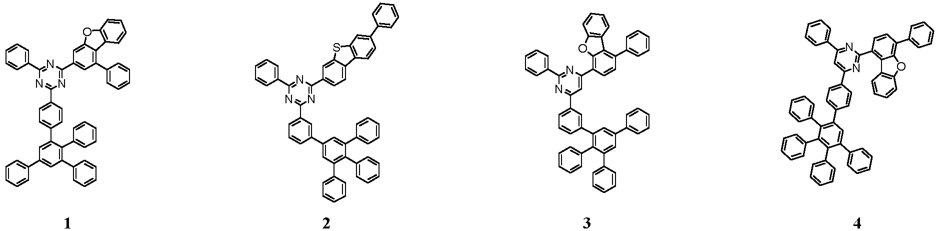

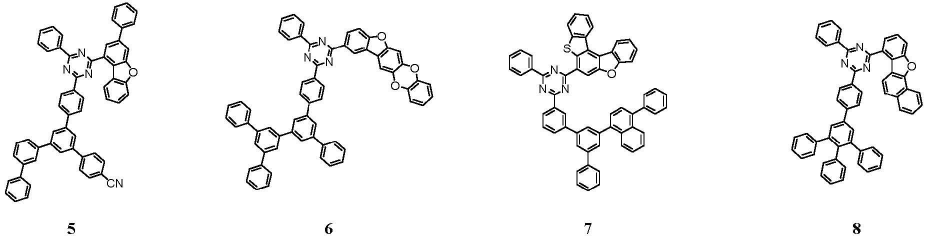

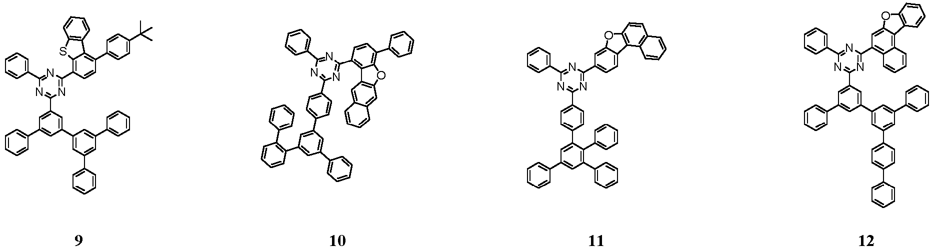

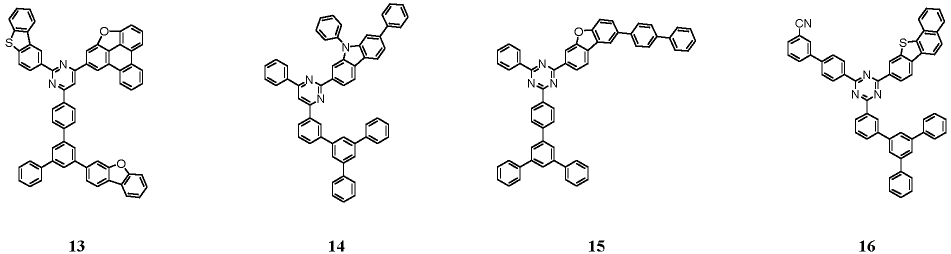

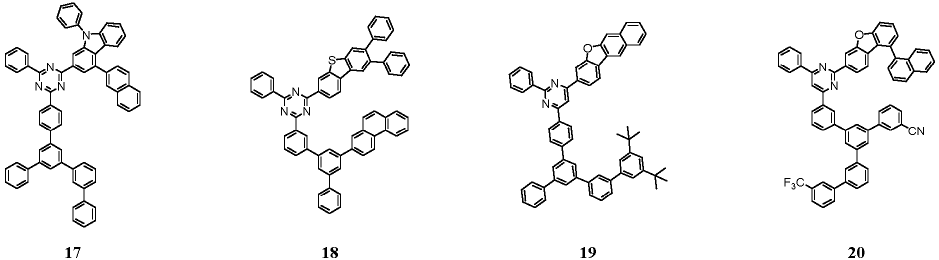

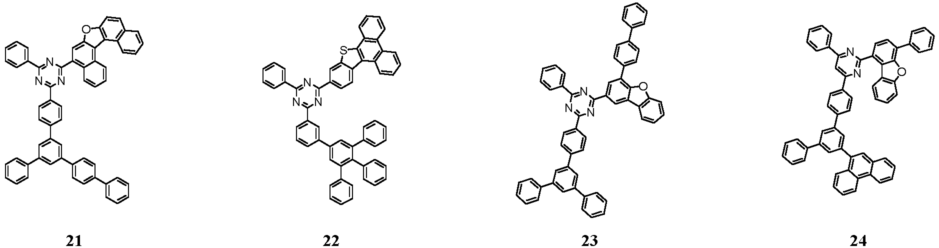

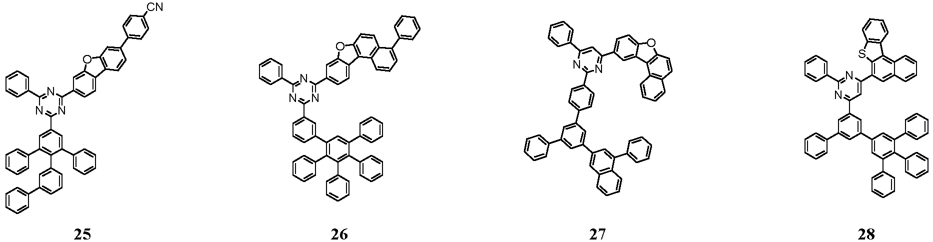

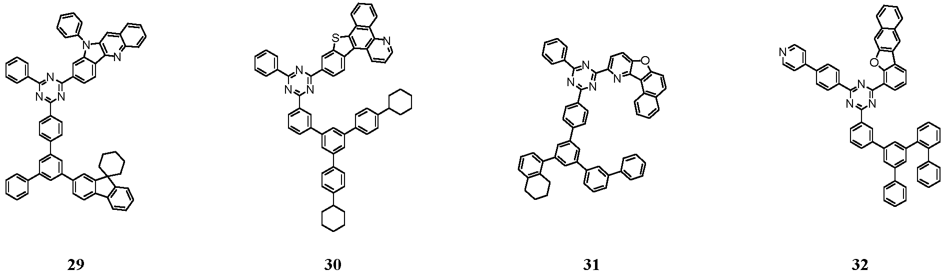

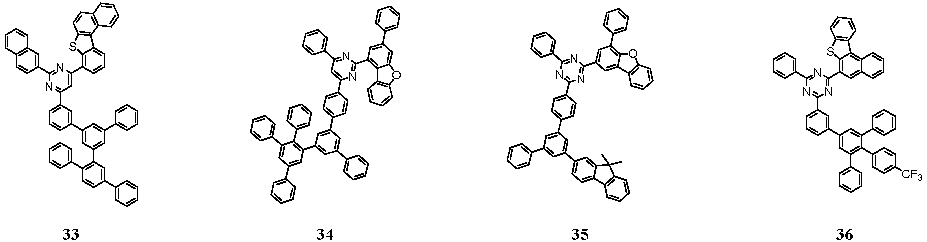

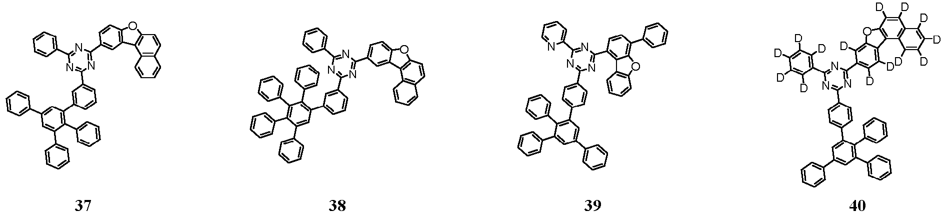

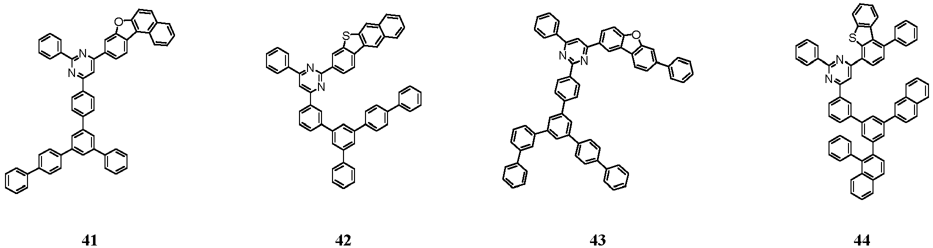

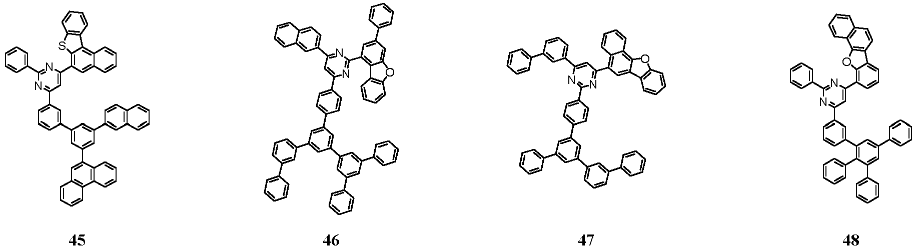

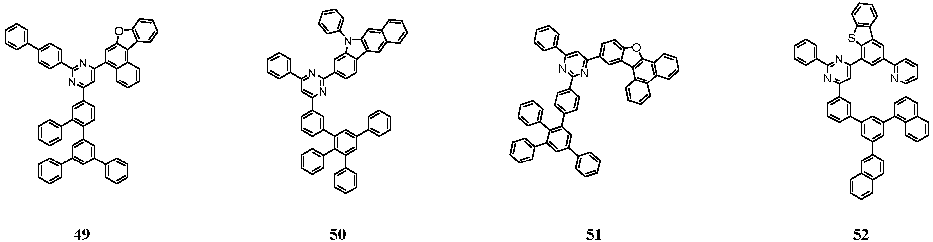

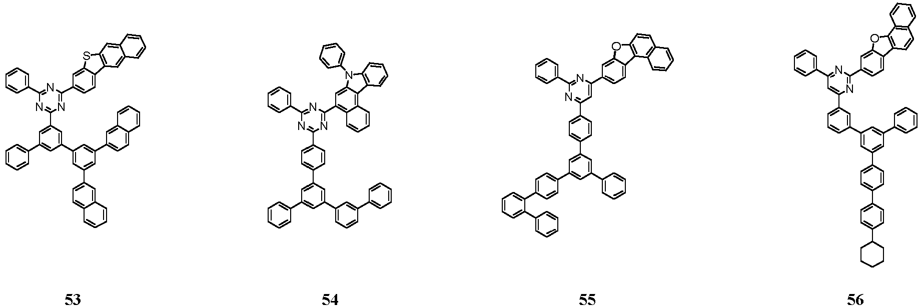

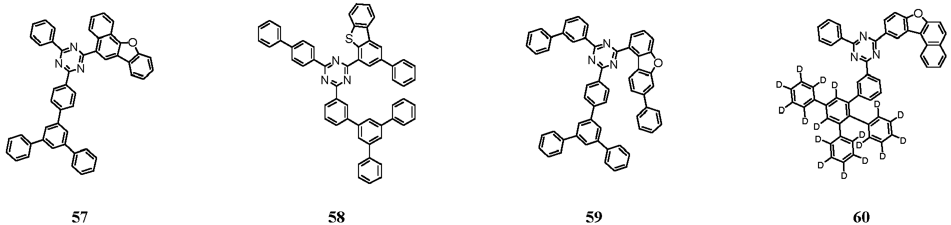

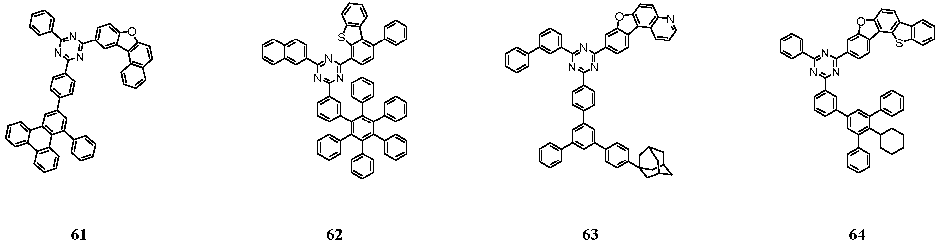

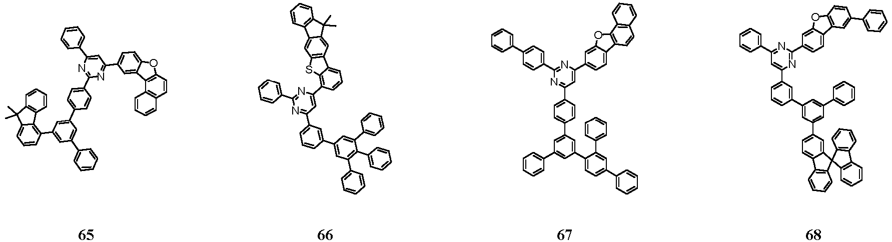

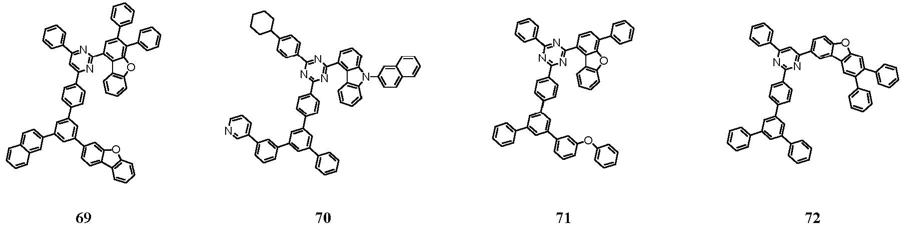

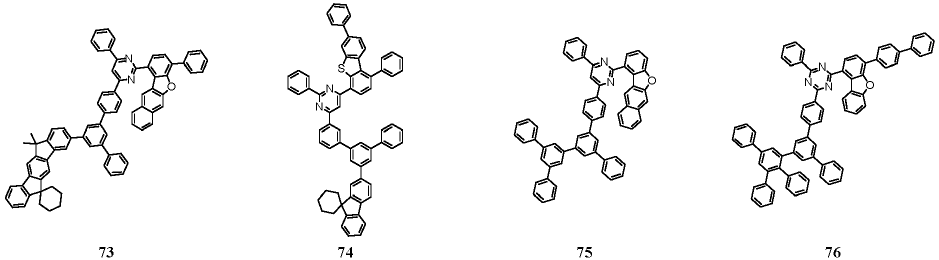

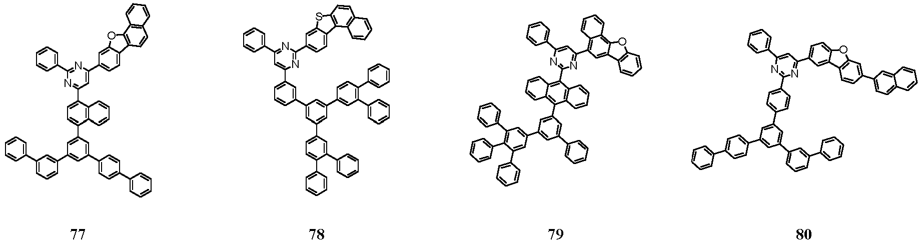

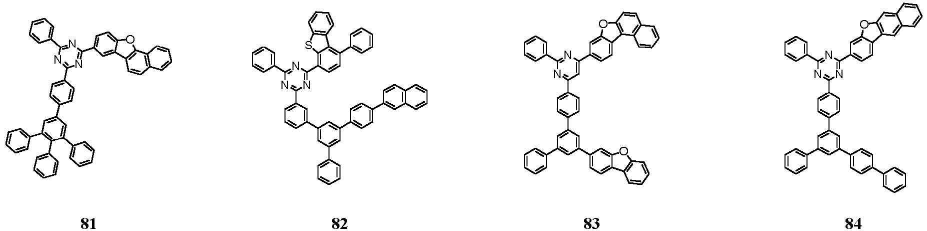

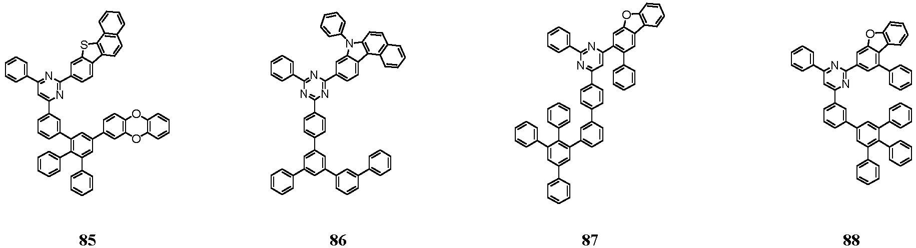

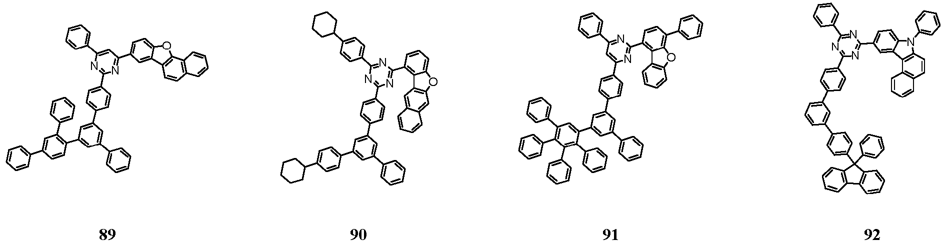

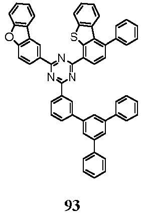

As used herein, "alkyl" refers to a monovalent substituent derived from a linear or branched chain saturated hydrocarbon having 1 to 40 carbon atoms. Examples of such alkyl may include, but not limited to, methyl, ethyl, propyl, isobutyl, sec-butyl, pentyl, iso-amyl, hexyl or the like.

As used herein, "alkenyl" refers to a monovalent substituent derived from a C₂ to C₄₀ linear or branched chain unsaturated hydrocarbon, having at least one carbon-carbon double bond. Examples of such alkenyl may include, but not limited to, vinyl, allyl, isopropenyl, 2-butenyl or the like.

As used herein, "alkynyl" refers to a monovalent substituent derived from a C₂ to C₄₀ linear or branched chain unsaturated hydrocarbon, having at least one carbon-carbon triple bond. Examples of such alkynyl may include, but not limited to, ethynyl, 2-propynyl or the like.

As used herein, "aryl" refers to a monovalent substituent derived from a C₆ to C₄₀ aromatic hydrocarbon having a structure with a single ring or two or more rings combined with each other. In addition, a form in which two or more rings are pendant (e.g., simply attached) to or fused with each other may also be included. Examples of such aryl may include, but not limited to, phenyl, naphthyl, phenanthryl, anthryl or the like.

As used herein, "heteroaryl" refers to a monovalent substituent derived from a monoheterocyclic or polyheterocyclic aromatic hydrocarbon having 5 to 40 nuclear atoms. In such an embodiment, one or more carbons in the ring, preferably one to three carbons, are substituted with a heteroatom such as N, O, S or Se. In addition, a form in which two or more rings are pendant to or fused with each other may be included and a form fused with an aryl group may be included. Examples of such heteroaryl may include, but not limited to, a 6-membered monocyclic ring such as pyridyl, pyrazinyl, pyrimidinyl, pyridazinyl and triazinyl; a polycyclic ring such as phenoxathienyl, indolizinyl, indolyl, purinyl, quinolyl, benzothiazole, and carbazolyl; 2-furanyl; N-imidazolyl; 2-isoxazolyl; 2-pyridinyl; 2-pyrimidinyl or the like.

As used herein, "aryloxy" is a monovalent substituent represented by RO-, where R refers to a C₅ to C₄₀ aryl. Examples of such aryloxy may include, but not limited to, phenyloxy, naphthyloxy, diphenyloxy or the like.

As used herein, "alkyloxy" refers to a monovalent substituent represented by R'O-, where R' refers to a C₁ to C₄₀ alkyl. Such alkyloxy may include a linear, branched or cyclic structure. Examples of such alkyloxy may include, but not limited to, methoxy, ethoxy, n-propoxy, 1-propoxy, t-butoxy, n-butoxy, pentoxy or the like.

As used herein, "arylamine" refers to amine substituted with a C₆ to C₄₀ aryl.

As used herein, "cycloalkyl" refers to a monovalent substituent derived from a C₃ to C₄₀ monocyclic or polycyclic non-aromatic hydrocarbon. Examples of such cycloalkyl may include, but not limited to, cyclopropyl, cyclopentyl, cyclohexyl, norbornyl, adamantine or the like.

As used herein, "heterocycloalkyl" refers to a monovalent substituent derived from a non-aromatic hydrocarbon having 3 to 40 nuclear atoms, where one or more carbons in the ring, preferably one to three carbons, are substituted with a heteroatom such as N, O, S or Se. Examples of such heterocycloalkyl may include, but not limited to, morpholine, piperazine or the like.

As used herein, "alkylsilyl" refers to silyl substituted with a C₁ to C₄₀ alkyl, and "arylsilyl" refers to silyl substituted with a C₅ to C₄₀ aryl.

As used herein, the term "fused ring (e.g., condensed ring)" refers to a condensed aliphatic ring, a condensed aromatic ring, a condensed heteroaliphatic ring, a condensed heteroaromatic ring, or a combination thereof.

### <Electron transport layer material>

The present invention provides an electron transport layer including the compound represented by Chemical Formula 1.

The electron transport layer (ETL) serves to move electrons injected from a cathode to an adjacent layer, specifically a light emitting layer.

The compound represented by Chemical Formula 1 may be used alone as an electron transport layer (ETL) material, or may be used in combination with an electron transport layer material known in the art. It may preferably be used alone.

The electron transport layer material that may be used in combination with the compound of Chemical Formula 1 may include an electron transport material commonly known in the art. Non-limiting examples of applicable electron transport materials may include oxazole-based compounds, isoxazole-based compounds, triazole-based compounds, isothiazole-based compounds, oxadiazole-based compounds, thiadiazole-based compounds, perylene-based compounds, aluminum complexes (e.g., tris(8-quinolinolato)-aluminium (Alq₃), BAlq, SAlq, Almq₃, gallium complexes (e.g., Gaq'2OPiv, Gaq'2OAc, 2(Gaq'2)), etc. These may be used alone or two or more types thereof may be used in combination.

In the present invention, when the compound of Chemical Formula 1 and the electron transport layer material are used in combination, a mixing ratio thereof is not particularly limited, and may be appropriately adjusted within a range known in the art.

### <Auxiliary electron transport layer material>

In addition, the present invention provides an auxiliary electron transport layer including the compound represented by Chemical Formula 1.

The auxiliary electron transport layer is disposed between the light emitting layer and the electron transport layer and serves to substantially prevent diffusion of excitons or holes generated in the light emitting layer into the electron transport layer.

The compound represented by Chemical Formula 1 may be used alone as an auxiliary electron transport layer material, or may be combined with an electron transport layer material known in the art. It may preferably be used alone.

The auxiliary electron transport layer material that may be used in combination with the compound of Chemical Formula 1 includes an electron transport material commonly known in the art. For example, the auxiliary electron transport layer may include an oxadiazole derivative, a triazole derivative, a phenanthroline derivative (e.g., BCP), a heterocyclic derivative containing nitrogen, and the like.

In the present invention, when the compound of Chemical Formula 1 and the auxiliary electron transport layer material are used in combination, a mixing ratio thereof is not particularly limited, and may be appropriately adjusted within a range known in the art.

### <Organic electroluminescent element>

Another aspect of the present invention is directed to an organic electroluminescent element ("organic EL element") including the compound represented by Chemical Formula 1.

More specifically, the organic EL element according to the present invention includes an anode (e.g., a positive electrode), a cathode (e.g., a negative electrode), and one or more organic layers disposed between the anode and the cathode, and at least one of the one or more organic layers includes the compound represented by Chemical Formula 1. In such an embodiment, the compound may be used alone or in combination of two or more kinds thereof.

The one or more organic layers may be any one or more of a hole injection layer, a hole transport layer, a light emitting layer, a light emitting auxiliary layer, a lifespan improvement layer, an electron transport layer, an auxiliary electron transport layer and an electron injection layer, and at least one of the organic layers may include the compound represented by Chemical Formula 1. Specifically, the organic layer including the compound represented by Chemical Formula 1 may preferably be a light emitting layer (more specifically, a phosphorescence emitting host material), an electron transport layer, and an auxiliary electron transport layer.

The light emitting layer of the organic EL element of the present invention includes a host material and a dopant material, and may include the compound of Chemical Formula 1 as a host material. In addition, the light emitting layer of the present invention may include, as a host, other compounds known in the art rather than or in addition to the compound of Chemical Formula 1.

When the compound represented by Chemical Formula 1 is included as a material for the light emitting layer of the organic EL element, preferably as a blue, green, or red phosphorescent host material, since a bonding force between holes and electrons in the light emitting layer is increased, the efficiency (luminescence efficiency and power efficiency), lifespan, luminance, driving voltage, and the like of the organic EL element may be improved. Specifically, the compound represented by Chemical Formula 1 may be preferably included in an organic EL element as a green and/or red phosphorescent host, a fluorescent host, or a dopant material. In particular, the compound represented by Chemical Formula 1 of the present invention may preferably be a green phosphorescent exciplex N-type host material for a high-efficiency light emitting layer.

A structure of the organic EL element of the present invention is not particularly limited, and may be, for example, a structure in which a substrate, an anode, a hole injection layer, a hole transport layer, a light emitting auxiliary layer, a light emitting layer, an electron transport layer, and a cathode are sequentially stacked. In such a case, at least one of the hole injection layer, the hole transport layer, the light emitting auxiliary layer, the light emitting layer, the electron transport layer and the electron injection layer may include the compound represented by Chemical Formula 1, and preferably, the light emitting layer, more preferably a phosphorescent host, may include the compound represented by Chemical Formula 1. Meanwhile, an electron injection layer may be additionally stacked on the electron transport layer.

The organic EL element of the present invention may have a structure in which an insulating layer or an adhesive layer is inserted at an interface between the electrode and the organic layer.

The organic EL element of the present invention may be prepared using materials and methods known in the art to form organic layers and electrodes, except that one or more layers of the aforementioned organic layers include the compound represented by Chemical Formula 1.

The organic layer may be formed by a vacuum deposition method or a solution coating method. Examples of the solution coating method may include, but not limited to, spin coating, dip coating, doctor blading, inkjet printing, thermal transfer or the like.

The substrate used in preparation of the organic EL element of the present invention is not particularly limited, and non-limiting examples thereof may include silicon wafers, quartz, glass plates, metal plates, plastic films, sheets or the like.

In addition, an anode material may use any anode material known in the art without limitation. Examples of the anode material may include, but not limited to, a metal such as vanadium, chromium, copper, zinc, and gold or an alloy thereof; metal oxides such as zinc oxide, indium oxide, indium tin oxide (ITO), or indium zinc oxide (IZO); combination of oxide with metal such as ZnO:Al or SnO₂:Sb; conductive polymers such as polythiophene, poly(3-methylthiophene), poly [3,4-(ethylene-1,2-dioxy) thiophene] (PEDT), polypyrrole or polyaniline; carbon black or the like.

In addition, a cathode material may use any cathode material known in the art without limitation. Examples of the cathode material may include, but not limited to, a metal such as magnesium, calcium, sodium, potassium, titanium, indium, yttrium, lithium, gadolinium, aluminum, silver, tin, or lead or an alloy thereof; a multi-layered material such as LiF/Al or LiO₂/Al or the like.

In addition, materials for the hole injection layer, the hole transport layer, the electron injection layer, and the electron transport layer are not particularly limited, and conventional materials known in the art may be used without limitation.

Hereinafter, the present invention will be described in detail through examples. However, the following examples are only to illustrate the present invention, and the present invention is not limited by the following examples.

### [Preparation Example 1]

### <Step 1> Synthesis of 3'-chloro-5'-phenyl-1,1':2',1''-terphenyl

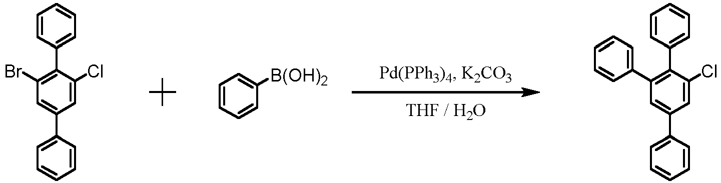

100 g (290.9 mmol) of 2'-bromo-6'-chloro-1,1':4',1"-terphenyl, 37.3 g (305.5 mmol) of phenylboronic acid, 10.1 g (8.7 mmol) of Pd(PPh₃)₄, and 120.6 g (872.98 mmol) of K₂CO₃ were added to 970 ml of THF and 240 ml of H₂O, and the mixture was heated under reflux with stirring for 6 hours. After the reaction was completed, the mixture was extracted with dichloromethane, and MgSO₄ was added to remove moisture, followed by filtering, and then the solvent of an organic layer was concentrated under reduced pressure, and the product was purified by column chromatography using dichloromethane and hexane. The purified product was solidified using methanol. The solid was filtered, washed with methanol, and dried in an oven to obtain the target compound, 3'-chloro-5'-phenyl-1,1':2',1"-terphenyl (75.6 g, yield 76.3%).
Mass : [(M+H)⁺] : 341

### <Step 2> Synthesis of Core 1

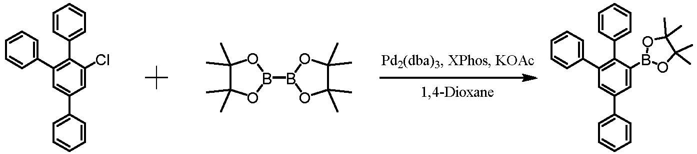

75.6 g (221.7 mmol) of 3'-chloro-5'-phenyl-1,1':2',1"-terphenyl, 73.2 g (288.3 mmol) of 4,4,4',4',5,5,5',5'-octamethyl-2,2'-bi(1,3,2-dioxaborolane), 6.1 g (6.7 mmol) of Pd₂(dba)₃, 65.3 g (665.4 mmol) of KOAc, 6.3 g (13.3 mmol) of Xphos were added to 750 ml of 1,4-Dioxane, and the mixture was heated under reflux for 12 hours. After the reaction was completed, the mixture was extracted with dichloromethane, and MgSO₄ was added to remove moisture, followed by filtering, and then the solvent of an organic layer was concentrated under reduced pressure, and the product was purified by column chromatography using dichloromethane and hexane. The purified product was solidified using methanol. The solid was filtered, washed with methanol, and dried in an oven to obtain the target compound, Core 1 (72.7g, yield 75.8%).
Mass : [(M+H)⁺] : 433

### [Preparation Example 2]

### <Step 1> Synthesis of 5'-chloro-1,1':3',1":3',1‴-quaterphenyl

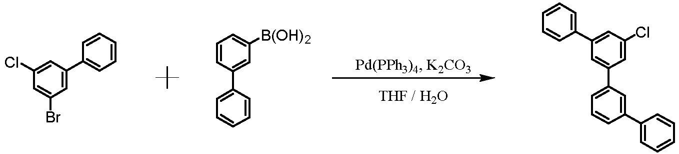

70 g (261.6 mmol) of 3-bromo-5-chloro-1,1'-biphenyl, 54.4 g (274.7 mmol) of [1,1'-biphenyl]-3-ylboronic acid, 9.1 g(7.8 mmol) of Pd(PPh₃)₄, and 108.5 g(784.9 mmol) of K₂CO₃ were added to 870 ml of THF 870 ml, and 220 ml of H₂O 220 ml, and the target compound, 5'-chloro-1,1':3',1'':3'',1‴-quaterphenyl (74.1 g, yield 83.1%) was obtained using the same manner as in Step 1 of Preparation Example 1.
Mass : [(M+H)⁺] : 341

### <Step 2> Synthesis of Core 2

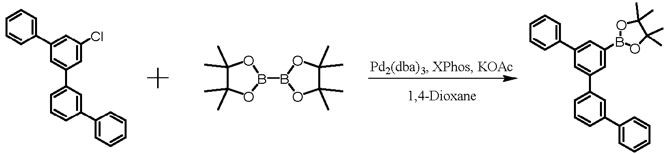

74.1 g (221.7 mmol) of 5'-chloro-1,1':3',1":3",1‴-quaterphenyl, 71.8 g (282.6 mmol) of 4,4,4',4',5,5,5',5'-octamethyl-2,2'-bi(1,3,2-dioxaborolane), 6.0 g (6.5 mmol) of Pd₂(dba)₃, 64.0 g (652.2 mmol) of KOAc, 6.2 g (13.0 mmol)of Xphos were added to 750 ml of 1,4-Dioxane 750 ml, and Core 2 (77.2 g, yield 77.1%) was obtained using the same manner as in the Core 1 synthesis method of Preparation Example 1.
Mass : [(M+H)+] : 433

### [Preparation Example 3]

### <Step 1> Synthesis of 5'-chloro-3'-phenyl-1,1':2',1"-terphenyl

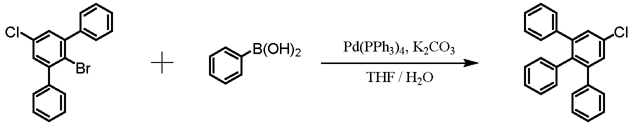

70 g (261.6 mmol) of 2'-bromo-5'-chloro-1,1':3',1"'-terphenyl, 54.4 g (274.7 mmol) of phenylboronic acid, 9.1 g (7.8 mmol) of Pd(PPh₃)₄, 108.5 g(784.9 mmol) of K₂CO₃ were added to 870 ml of THF, 220 ml of H₂O, and 5'-chloro-3'-phenyl-1,1':2',1"-terphenyl (47.5 g, yield 68.4%) was obtained using the same manner as in Step 1 of Preparation Example 1.

### <Step 2> Synthesis of Core 3

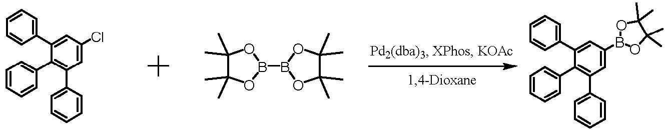

47.5 g (139.3 mmol) of 5'-chloro-3'-phenyl-1,1':2',1"-terphenyl, 45.9 g (181.1 mmol) of 4,4,4',4',5,5,5',5'-octamethyl-2,2'-bi(1,3,2-dioxaborolane), 3.8 g (4.2 mmol) of Pd₂(dba)₃, 41.0 g (417.9 mmol) of KOAc, 4.0 g (8.3 mmol) of Xphos were added to 470 ml of 1,4-Dioxane, and Core 3 (45.25 g, yield 75.1%) was obtained using the same manner as in the Core 1 synthesis method of Preparation Example 1.
Mass : [(M+H)⁺] : 433

### [Preparation Example 4]

### <Synthesis 1> Synthesis of 5 "-chloro-1,1' :3',1":3",1‴:4‴,1ʺʺ-quinquephenyl

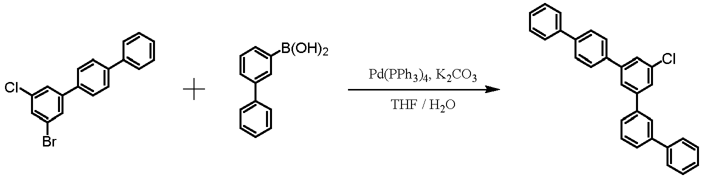

50 g (145.5 mmol) of 3-bromo-5-chloro-1,1':4',1"-terphenyl, 30.2 g (152.8 mmol) of [1,1'-biphenyl]-3-ylboronic acid, 5.0 g (4.4 mmol) of Pd(PPh₃)₄, and 60.3 g (436.5 mmol) of K₂CO₃ were added to 490 ml of THF 490 ml, 125 ml of H₂O, and 5''-chloro-1,1':3',1":3'',1‴:4‴,1ʺʺ-quinquephenyl (53.9 g, yield 88.9%) was obtained using the same manner as in Step 1 of Preparation Example 1.
Mass : [(M+H)⁺] : 417

### <Step 2> Synthesis of Core 4

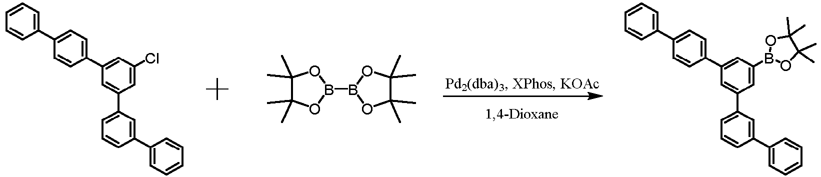

53.9 g (129.3 mmol) of 5''-chloro-1,1':3',1'':3'',1‴:4‴,1ʺʺ-quinquephenyl, 45.9 g (181.1 mmol) of 4,4,4',4',5,5,5',5'-octamethyl-2,2'-bi(1,3,2-dioxaborolane), 3.6 g (3.9 mmol) of Pd₂(dba)₃, 38.1 g (388.0 mmol) of KOAc, and 3.7 g (7.8 mmol) of Xphos were added to 430 ml of 1,4-Dioxane 430 ml, Core 4 (41.96 g, yield 63.8%) was obtained using the same manner as in the Core 1 synthesis method of Preparation Example 1.
Mass : [(M+H)⁺] : 508

### [Preparation Example 5]

### <Step 1> Synthesis of 2-(5'-chloro-[1,1':3',1''-terphenyl]-4-yl)naphthalene

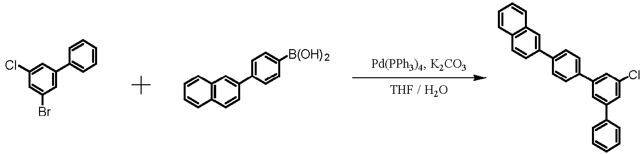

50 g (186.9 mmol) of 3-bromo-5-chloro-1,1'-biphenyl, 48.9 g (196.2 mmol) of (4-(naphthalen-2-yl)phenyl)boronic acid, 6.5 g(5.6 mmol) of Pd(PPh₃)₄, and 77.5 g(560.6 mmol) of K₂CO₃ were added to 625 ml of THF, 155 ml of H₂O, and 2-(5'-chloro-[1,1':3',1"-terphenyl]-4-yl)naphthalene (55.9 g, yield 76.6%) was obtained using the same manner as in Step 1 of Preparation Example 1.
Mass : [(M+H)⁺] : 391

### <Step 2> Synthesis of Core 5

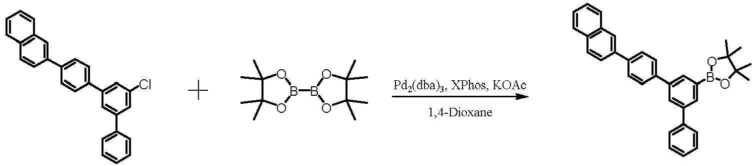

55.9 g (143.1 mmol) of 2-(5'-chloro-[1,1':3',1"-terphenyl]-4-yl)naphthalene, 47.2 g (186.1 mmol) of 4,4,4',4',5,5,5',5'-octamethyl-2,2'-bi(1,3,2-dioxaborolane), 3.9 g(4.3 mmol) of Pd₂(dba)₃, 42.1 g (429.4 mmol) of KOAc, and 4.1 g (8.6 mmol) of Xphos were added to 480 ml of 1,4-Dioxane, and Core 4 (40.26 g, yield 58.3%) was obtained using the same manner as in the Core 1 synthesis method of Preparation Example 1.
Mass : [(M+H)⁺] : 483

### [Preparation Example 6]

### <Step 1> Synthesis of 3-(5-chloro-[1,1'-biphenyl]-3-yl)dibenzo[b,d]furan

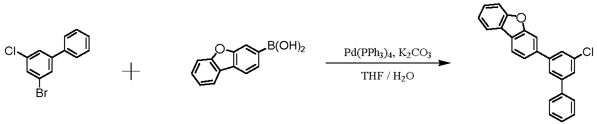

50 g (186.9 mmol) of 3-bromo-5-chloro-1,1'-biphenyl, 41.6 g (196.2 mmol) of dibenzo[b,d]furan-3-ylboronic acid, 6.5 g (5.6 mmol) of Pd(PPh₃)₄, and 77.5 g(560.6 mmol) of K₂CO₃ were added to 625 ml of THF, 155 ml of H₂O, and 3-(5-chloro-[1,1'-biphenyl]-3-yl)dibenzo[b,d]furan (56.4 g, yield 85.1%) was obtained using the same manner as in Step 1 of Preparation Example 1.
Mass : [(M+H)⁺] : 355

### <Step 2> Synthesis of Core 6

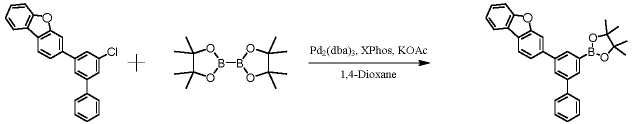

56.4 g(159.0 mmol) of 3-(5-chloro-[1,1'-biphenyl]-3-yl)dibenzo[b,d]furan, 52.5 g(206.7 mmol) of 4,4,4',4',5,5,5',5'-octamethyl-2,2'-bi(1,3,2-dioxaborolane), 4.4 g(4.7 mmol) of Pd₂(dba)₃, 46.8 g (477.1 mmol) of KOAc, and 4.5 g (9.5 mmol) of Xphos were added to 530 ml of 1,4-Dioxane, and Core 6 (56.7 g, yield 79.9%) was obtained using the same manner as in the Core 1 synthesis method of Preparation Example 1.
Mass : [(M+H)⁺] : 447

### [Synthesis Example 11 Synthesis of Compound 1

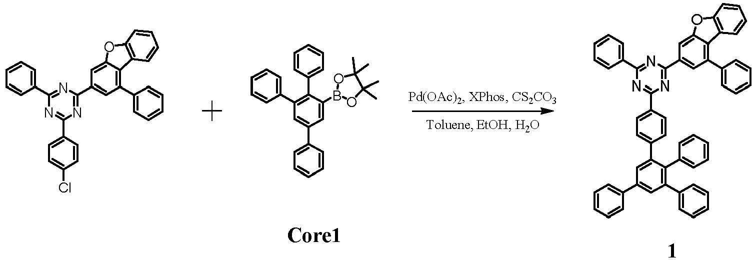

15.0 g (1eq, 29.4 mmol) of 2-(4-chlorophenyl)-4-phenyl-6-(1-phenyldibenzo[b,d]furan-3-yl)-1,3,5-triazine, 13.4 g(1.05eq, 30.9 mmol) of Core 1 in Preparation Example 1, 0.2 g(0.03eq, 0.9 mmol) of Pd(OAc)₂, 28.7 g(3.0eq, 88.2 mmol) of Cs₂CO₃, and 0.8 g(0.06eq, 1.8 mmol) of Xphos were added to 100 ml of Toluene, 25 ml of EtOH, and 25 ml of H₂O, and the mixture was heated under reflux for 12 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 1 (15.8 g, yield 68.8%) .
Mass : [(M+H)⁺] : 780

### [Synthesis Example 2] Synthesis of Compound 3

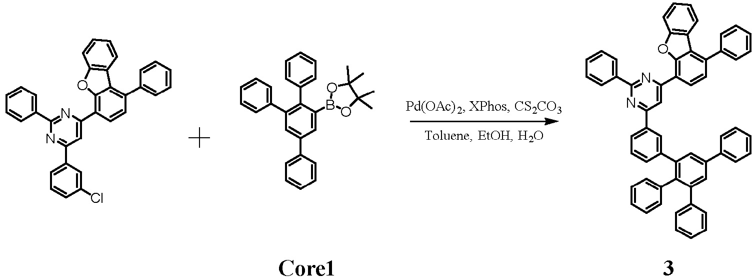

Except that 15.0 g (29.5 mmol) of 4-(3-chlorophenyl)-2-phenyl-6-(1-phenyldibenzo[b,d]furan-4-yl)pyrimidine was used instead of 2-(4-chlorophenyl)-4-phenyl-6-(1-phenyldibenzo[b,d]furan-3-yl)-1,3,5-triazine, the remaining reagents were used in the same equivalent ratio as in [Synthesis Example 1], and the same process was performed to obtain the target compound, Compound 3 (12.7g, yield 55.2%).
Mass : [(M+H)⁺] : 779

### [Synthesis Example 3] Synthesis of Compound 11

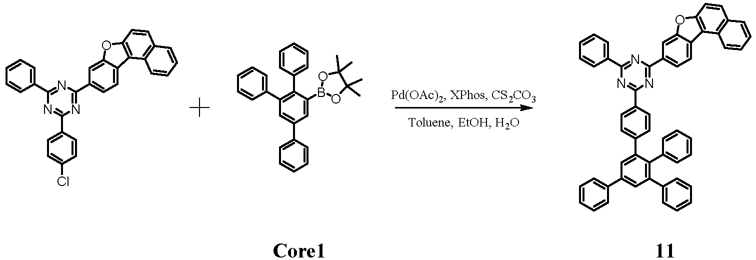

Except that 15.0 g(30.9 mmol) of 2-(4-chlorophenyl)-4-(naphtho[2,1-b]benzofuran-9-yl)-6-phenyl-1,3,5-triazine was used instead of 2-(4-chlorophenyl)-4-phenyl-6-(1-phenyldibenzo[b,d]furan-3-yl)-1,3,5-triazine, the remaining reagents were used in the same equivalent ratio as in [Synthesis Example 1], and the same process was performed to obtain the target compound, Compound 11 (19.0g, yield 81.4%).
Mass : [(M+H)⁺] : 754

### [Synthesis Example 4] Synthesis of Compound 16

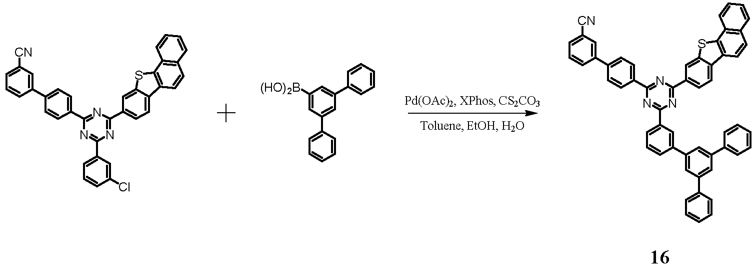

10.0 g(1eq, 16.6 mmol) of 4'-(4-(benzo[b]naphtho[2,1-d]thiophen-9-yl)-6-(3-chlorophenyl)-1,3,5-triazin-2-yl) -[1, 1'-biphenyl]-3-carbonitrile, 4.78 g(1.05eq, 17.4 mmol) of [1,1':3',1"-terphenyl]-5'-ylboronic acid, 0.1 g(0.03eq, 0.5 mmol) of Pd(OAc)₂, 16.26 g(3.0eq, 49.9 mmol) of Cs₂CO₃, and 0.5 g(0.06eq, 1.0 mmol) of Xphos were added to 130 ml of Toluene, 30 ml of EtOH, and 30 ml of H₂O, and the mixture was heated under reflux for 8 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 16 (16 5.7 g, yield 42.8 %).
Mass : [(M+H)⁺] : 795

### [Synthesis Example 5] Synthesis of Compound 17

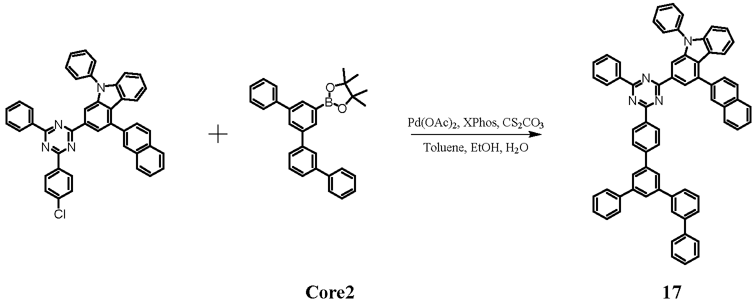

15.0 g(1eq, 23.6 mmol) of 2-(4-(4-chlorophenyl)-6-phenyl-1,3,5-triazin-2-yl)-4-(naphthalen-2-yl)-9-phenyl-9H-carbazole, 10.7 g(1.05eq, 24.8 mmol) of Core 2 in Preparation Example 2, 0.16 g(0.03eq, 0.7 mmol) of Pd(OAc)₂, 23.1 g(3.0eq, 70.8 mmol) of Cs₂CO₃, and 0.67 g(0.06eq, 1.4 mmol) of Xphos were added to 100 ml of Toluene, 25 ml of EtOH, and 25 ml of H₂O, and the mixture was heated under reflux for 5 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 17 (10.9 g, yield 51.2%).
Mass : [(M+H)⁺] : 906

### [Synthesis Example 6] Synthesis of Compound 28

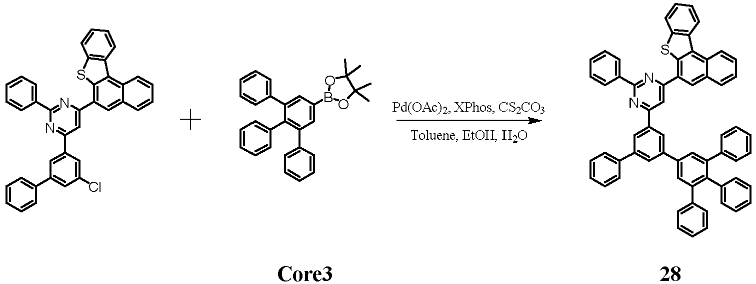

15.0 g(1eq, 26.08 mmol) of 4-(benzo[b]naphtho[1,2-*d*]thiophen-6-yl)-6-(5-chloro-[1,1'-biphenyl]-3-yl)-2-phenylpyrimidine, 11.8 g(1.05eq, 27.39 mmol) of Core 3 in Preparation Example 3, 0.18 g(0.03eq, 0.8 mmol) of Pd(OAc)₂, 25.5 g(3.0eq, 78.2 mmol) of Cs₂CO₃, and 0.74 g(0.06eq, 1.6 mmol) of Xphos were added to 120 ml of Toluene, 30 ml of EtOH, and 30 ml of H₂O, and the mixture was heated under reflux for 8 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 28 (15.7 g, yield 71.1%).
Mass : [(M+H)⁺] : 845

### [Synthesis Example 7] Synthesis of Compound 37

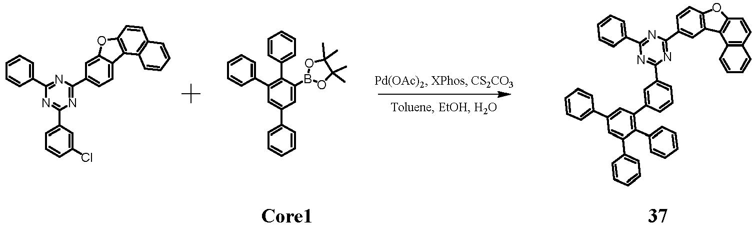

Except that 15.0 g(30.9 mmol) of 2-(4-chlorophenyl)-4-(naphtho[2,1-*b*]benzofuran-9-yl)-6-phenyl-1,3,5-triazine was used instead of 2-(4-chlorophenyl)-4-phenyl-6-(1-phenyldibenzo[*b,d*]furan-3-yl)-1,3,5-triazine, the remaining reagents were used in the same equivalent ratio as in [Synthesis Example 1], and the same process was performed to obtain the target compound, Compound 37 (17.8g, yield 76.5%).
Mass : [(M+H)⁺] : 754

### [Synthesis Example 8] Synthesis of Compound 49

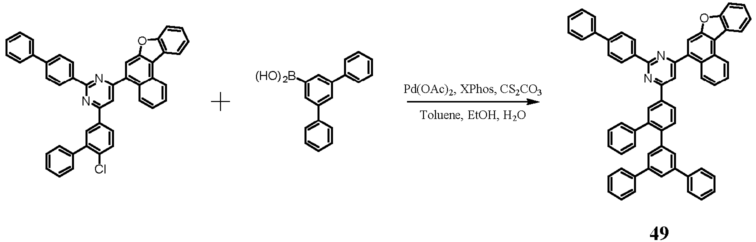

13.0 g(1eq, 20.5 mmol) of 2-([1,1'-biphenyl]-4-yl)-4-(6-chloro-[1,1'-biphenyl]-3-yl)-6-(naphtho[2,1-*b*]benzofuran-5-yl)pyrimidine, 5.9 g(1.05eq, 21.5 mmol) of [1,1':3',1''-terphenyl]-5'-ylboronic acid, 0.14 g(0.03eq, 0.6 mmol) of Pd(OAc)₂, 20.0 g(3.0eq, 61.4 mmol) of Cs₂CO₃, and 0.6 g(0.06eq, 1.2 mmol) of Xphos were added to 130 ml of Toluene, 30 ml of EtOH, and 30 ml of H₂O, and the mixture was heated under reflux for 10 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 49 (8.3 g, yield 48.9%).
Mass : [(M+H)⁺] : 830

### [Synthesis Example 9] Synthesis of Compound 50

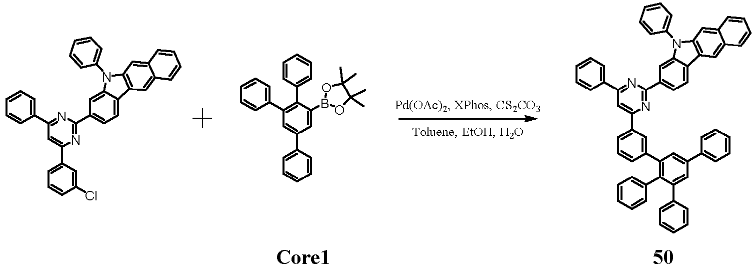

Except that 14.0 g(25.1 mmol) of 3-(4-(3-chlorophenyl)-6-phenylpyrimidin-2-yl)-5-phenyl-5H-benzo[*b*]carbazole was used instead of 2-(4-chlorophenyl)-4-phenyl-6-(1-phenyldibenzo[*b,d*]furan-3-yl)-1,3,5-triazine, the remaining reagents were used in the same equivalent ratio as in [Synthesis Example 1], and the same process was performed to obtain the target compound, Compound 50 (11.1g, yield 53.4%).
Mass : [(M+H)⁺] : 829

### [Synthesis Example 10] Synthesis of Compound 58

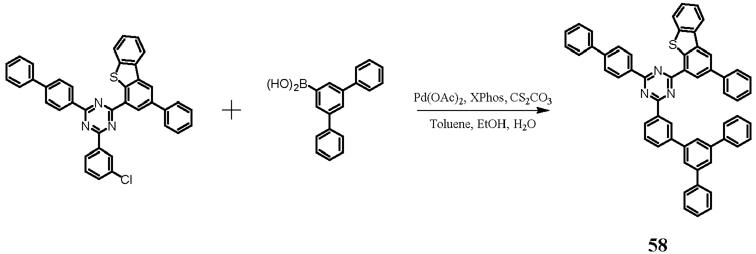

Except that 14.0 g(25.1 mmol) of 2-([1,1'-biphenyl]-4-yl)-4-(3-chlorophenyl)-6-(2-phenyldibenzo[*b,d*]thiophen-4-yl)-1,3,5-triazine was used instead of 2-([1,1'-biphenyl]-4-yl)-4-(6-chloro-[1,1'-biphenyl]-3-yl)-6-(naphtho[2,1-*b*]benzofuran-5-yl)pyrimidine, the remaining reagents were used in the same equivalent ratio as in [Synthesis Example 8], and the same process was performed to obtain the target compound, Compound 58 (12.4g, yield 62.5%).
Mass : [(M+H)⁺] : 797

### [Synthesis Example 11] Synthesis of Compound 72

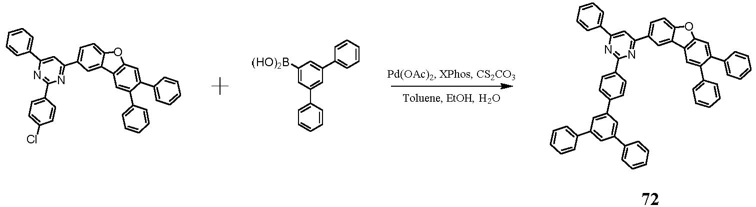

Except that 14.0 g(25.1 mmol) of 2-(4-chlorophenyl)-4-(7,8-diphenyldibenzo[b,d]furan-2-yl)-6-phenylpyrimidine was used instead of 2-([1,1'-biphenyl]-4-yl)-4-(6-chloro-[1,1'-biphenyl]-3-yl)-6-(naphtho[2,1-b]benzofuran-5-yl)pyrimidine, the remaining reagents were used in the same equivalent ratio as in [Synthesis Example 8], and the same process was performed to obtain the target compound, Compound 72 (14.4g, yield 72.1%).
Mass : [(M+H)⁺] : 799

### [Synthesis Example 12] Synthesis of Compound 80

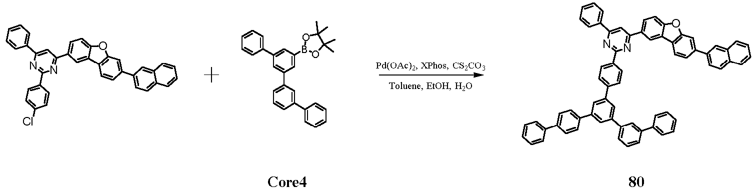

12.0 g(1eq, 21.5 mmol) of 2-(4-chlorophenyl)-4-(7-(naphthalen-2-yl)dibenzo[b,d]furan-2-yl)-6-phenylpyrimidine, 9.7 g(1.05eq, 22.5 mmol) of Core 4 in Preparation Example 4, 0.14 g(0.03eq, 0.6 mmol) of Pd(OAc)₂, 20.0 g(3.0eq, 64.4 mmol) of Cs₂CO₃, and 0.61 g(0.06eq, 1.3 mmol) of Xphos were added to 140 ml of Toluene, 35 ml of EtOH, and 35 ml of H₂O, and the mixture was heated under reflux for 8 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 80 (10.1 g, yield 51.8%).
Mass : [(M+H)⁺] : 906

### [Synthesis Example 13] Synthesis of Compound 81

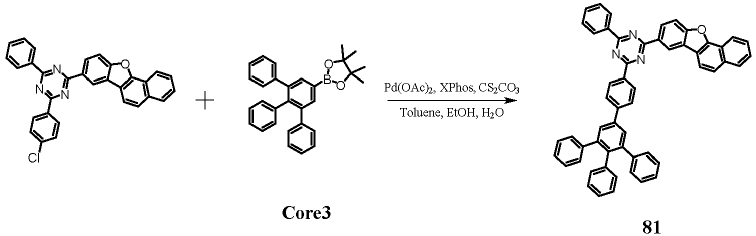

Except that 15.0 g(31.0 mmol) of 2-(4-chlorophenyl)-4-(naphtho[1,2-*b*]benzofuran-8-yl)-6-phenyl-1,3,5-triazine was used instead of 4-(benzo[*b*]naphtho[1,2-*d*]thiophen-6-yl)-6-(5-chloro-[1,1'-biphenyl]-3-yl)-2-phenylpyrimidine, the remaining reagents were used in the same equivalent ratio as in [Synthesis Example 6], and the same process was performed to obtain the target compound, Compound 81 (19.2g, yield 82.2%).
Mass : [(M+H)⁺] : 755

### [Synthesis Example 14] Synthesis of Compound 82

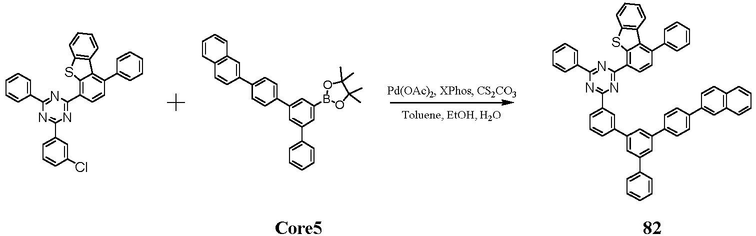

13.0 g(1eq, 24.7 mmol) of 2-(3-chlorophenyl)-4-phenyl-6-(1-phenyldibenzo[b,d]thiophen-4-yl)-1,3,5-triazine, 12.5 g(1.05eq, 25.9 mmol) of Core 5 in Preparation Example 5, 0.17 g(0.03eq, 0.67 mmol) of Pd(OAc)₂, 24.1 g(3.0eq, 74.1 mmol) of Cs₂CO₃, and 0.7 g(0.06eq, 1.5 mmol) of Xphos were added to 150 ml of Toluene, 40 ml of EtOH, and 40 ml of H₂O, and the mixture was heated under reflux for 8 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 82 (13.2 g, yield 63.3%).
Mass : [(M+H)⁺] : 847

### [Synthesis Example 15] Synthesis of Compound 83

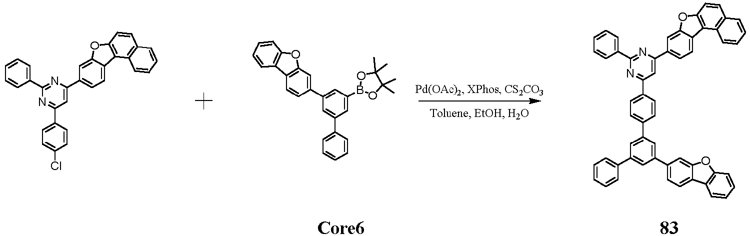

15.0 g(1eq, 31.1 mmol) of 4-(4-chlorophenyl)-6-(naphtho[2,1-*b*]benzofuran-9-yl)-2-phenylpyrimidine, 14.6 g(1.05eq, 25.9 mmol) of Core 6 in Preparation Example 6, 0.21 g(0.03eq, 0.93 mmol) of Pd(OAc)₂, 30.4 g(3.0eq, 93.2 mmol) of Cs₂CO₃, and 0.9 g(0.06eq, 1.9 mmol) of Xphos were added to 150 ml of Toluene, 40 ml of EtOH, and 40 ml of H₂O, and the mixture was heated under reflux for 10 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 83 (19.5 g, yield 81.9%).
Mass : [(M+H)⁺] : 768

### [Examples 1 to 10] Manufacturing of Blue organic EL elements

The compounds 1 to 93 were subjected to high-purity sublimation purification in a conventionally known method, and then blue organic EL elements were manufactured according to the following procedure.

A glass substrate thin-film-coated with indium tin oxide (ITO) to a thickness of 1,200 Å was washed with distilled water ultrasonically. After washing with distilled water was completed, the glass substrate was ultrasonically washed with a solvent, such as isopropyl alcohol, acetone and methanol, dried, transferred to a UV OZONE cleaner (Power sonic 405, Hwasin Tech), cleaned for 5 minutes using UV, and then transferred to a vacuum evaporator.

On the ITO transparent electrode prepared as above, HT-1 + 2% HAT-CN (100 Å) / HT-1 (1400 Å ) / HT-2 (50 Å ) / BH + 2% BD (200 Å ) / ET-2 (50 Å ) / each of compounds 1 to 87 : LiQ = 1:1 (300 Å ) / LiF (10 Å ) / Al (1000 Å ) were stacked in order to manufacture an organic EL element.

In such a case, the structures of HT-1, HAT-CN, HT-2, BH, BD, ET-1, ET-2 and LiQ are each as follows.

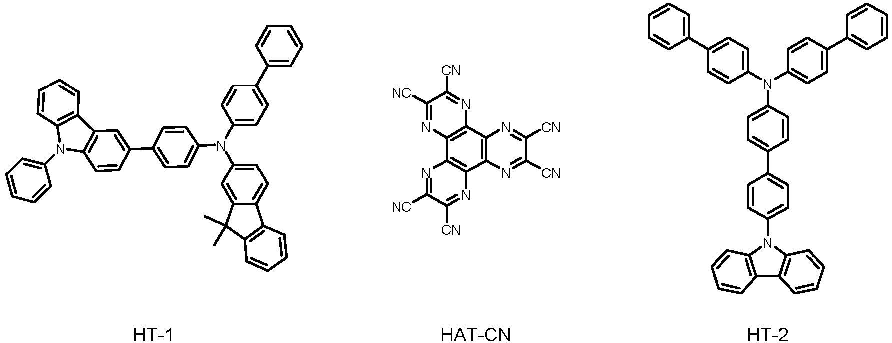

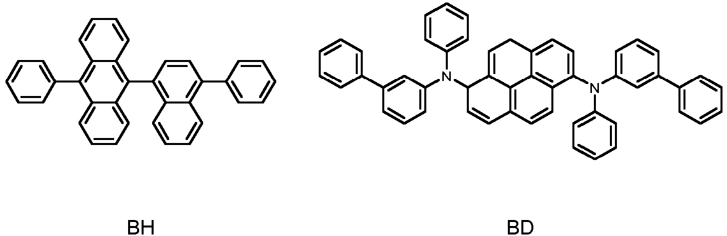

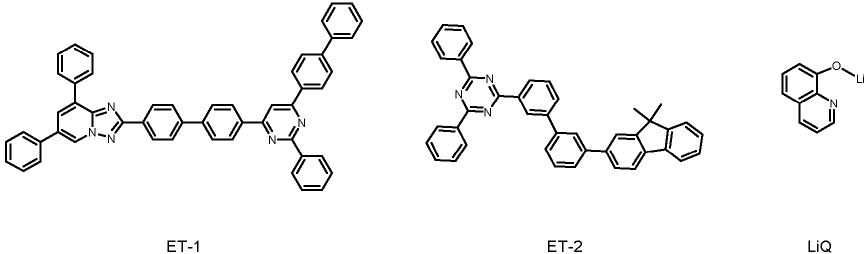

### [Comparative Example 1] Manufacturing of blue organic EL element

A blue organic EL element of Comparative Example 1 was manufactured in the same manner as in Example 1, except that Compound ET-1 was deposited at 300 Å instead of Compound 1 to 87 used as the electron transport layer material in Example 1.

### [Evaluation example 1]

For each of the blue organic EL elements manufactured in Examples (Ex.) 1 to 10 and Comparative Example (Comp. Ex.) 1, a driving voltage, an emission peak, and a current efficiency at a current density of 10 mA/cm² were measured, and the results are shown in Table 1 below.

**[Table 1]**

| Sample | Electron transport layer material | Driving voltage (V) | EL peak (nm) | Current efficiency (cd/A) |
|---|---|---|---|---|
| Ex. 1 | Compound 1 | 4.2 | 461 | 6.3 |
| Ex. 2 | Compound 3 | 4.3 | 459 | 6.4 |
| Ex. 3 | Compound 11 | 4.7 | 460 | 6.2 |
| Ex. 4 | Compound 16 | 4.3 | 460 | 6.1 |
| Ex. 5 | Compound 17 | 4.2 | 459 | 6.3 |
| Ex. 6 | Compound 18 | 4.7 | 458 | 5.9 |
| Ex. 7 | Compound 24 | 4.3 | 459 | 6.5 |
| Ex. 8 | Compound 37 | 4.5 | 461 | 6.0 |
| Ex. 9 | Compound 86 | 4.6 | 458 | 6.1 |
| Ex. 10 | Compound 87 | 4.3 | 460 | 6.5 |
| Comp. Ex. 1 | ET-1 | 4.8 | 460 | 5.8 |

As shown in Table 1, it was appreciated that the blue organic EL elements of Examples 1 to 10 using the compound according to the present invention as an electron transport layer material exhibit significantly excellent performance in terms of current efficiency, emission peak and driving voltage compared to the blue organic EL element of Comparative Example 1 using conventional compound ET-1 as an electron transport layer material.

### [Examples 11 to 33] Manufacturing of Blue organic EL elements

The compounds 1 to 93 were subjected to high-purity sublimation purification in a conventionally known method, and then blue organic EL elements were manufactured according to the following procedure.

A glass substrate thin-film-coated with indium tin oxide (ITO) to a thickness of 1,200 Å was washed with distilled water ultrasonically. After washing with distilled water was completed, the glass substrate was ultrasonically washed with a solvent, such as isopropyl alcohol, acetone and methanol, dried, transferred to a UV OZONE cleaner (Power sonic 405, Hwasin Tech), cleaned for 5 minutes using UV, and then transferred to a vacuum evaporator.

On the ITO transparent electrode prepared as above, HT-1 + 2% HAT-CN (100 Å ) / HT-1 (1400 Å ) / HT-2 (50 Å ) / BH + 2% BD (200 Å ) / each of compounds 1 to 93 (50 Å ) / ET-1 : LiQ = 1:1 (300 Å ) / LiF (10 Å ) / Al (1000 Å) were stacked in order to manufacture an organic EL element.

In such a case, the structures of HT-1, HAT-CN, HT-2, BH, BD, ET-1, ET-2 and LiQ are the same as described in Examples 1 to 10.

### [Comparative Examples 2 to 5] Manufacturing of blue organic EL element

A blue organic EL element of Comparative Examples 2 to 5 were manufactured in the same manner as in Example 11, except that Compounds ET-2 to ET-6 was deposited at 50 Å instead of Compound 1 to 93 used as the auxiliary electron transport layer material in Example 11.

In such a case, the structures of ET-2 to ET-6 are each as follows.

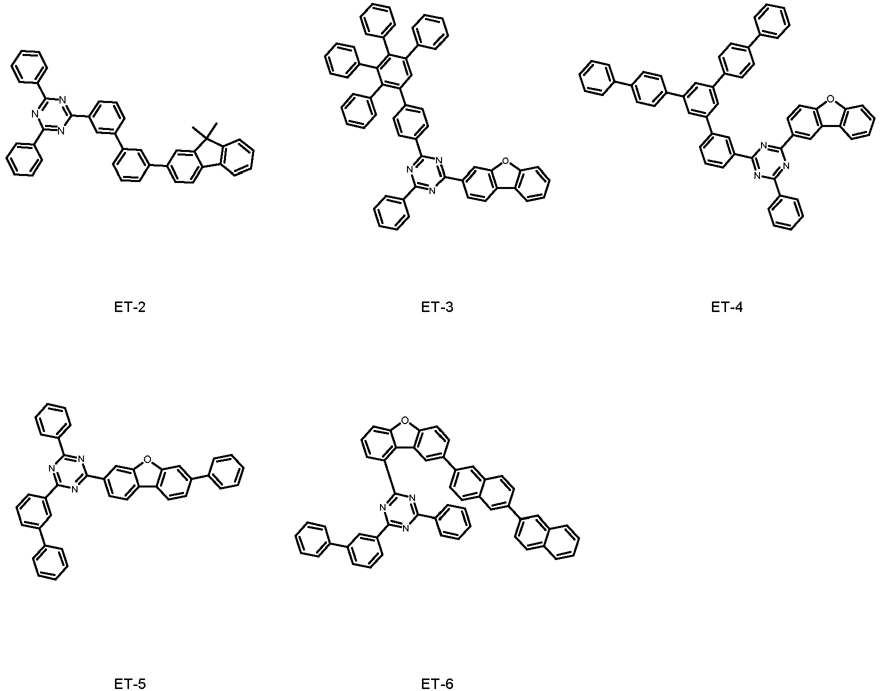

### [Evaluation example 2]

For each of the blue organic EL elements manufactured in Examples (Ex.) 11 to 33 and Comparative Examples (Comp. Ex.) 2 to 6, a driving voltage, an emission peak, and a current efficiency at a current density of 10 mA/cm² were measured, and the results are shown in Table 2 below.

**[Table 2]**

| Sample | Auxiliary electron transport layer material | Driving voltage (V) | EL peak (nm) | Current efficiency (cd/A) |
|---|---|---|---|---|
| Ex. 11 | Compound 1 | 3.7 | 460 | 7.2 |
| Ex. 12 | Compound 3 | 3.8 | 461 | 7.1 |
| Ex. 13 | Compound 4 | 4.2 | 460 | 6.9 |
| Ex. 14 | Compound 8 | 4.4 | 461 | 6.7 |
| Ex. 15 | Compound 11 | 3.9 | 460 | 6.9 |
| Ex. 16 | Compound 15 | 3.9 | 460 | 6.8 |
| Ex. 17 | Compound 24 | 4.1 | 458 | 6.7 |
| Ex. 18 | Compound 28 | 4.3 | 460 | 6.2 |
| Ex. 19 | Compound 35 | 3.9 | 460 | 7.1 |
| Ex. 20 | Compound 37 | 3.7 | 459 | 7.1 |
| Ex. 21 | Compound 38 | 3.7 | 460 | 6.9 |
| Ex. 22 | Compound 48 | 4.3 | 458 | 6.3 |
| Ex. 23 | Compound 49 | 4.5 | 461 | 6.0 |
| Ex. 24 | Compound 51 | 4.1 | 459 | 6.7 |
| Ex. 25 | Compound 54 | 4.2 | 458 | 6.3 |
| Ex. 26 | Compound 59 | 4.6 | 458 | 5.8 |
| Ex. 27 | Compound 61 | 4.3 | 457 | 6.1 |
| Ex. 28 | Compound 65 | 4.0 | 460 | 6.8 |
| Ex. 29 | Compound 81 | 3.7 | 458 | 6.7 |
| Ex. 30 | Compound 83 | 3.9 | 461 | 7.2 |
| Ex. 31 | Compound 86 | 4.3 | 460 | 6.4 |
| Ex. 32 | Compound 87 | 3.8 | 461 | 7.2 |
| Ex. 33 | Compound 93 | 4.1 | 457 | 6.7 |
| Comp. Ex. 2 | ET-2 | 4.4 | 457 | 6.2 |
| Comp. Ex. 3 | ET-3 | 4.6 | 461 | 5.9 |
| Comp. Ex. 4 | ET-4 | 4.6 | 460 | 6.0 |
| Comp. Ex. 5 | ET-5 | 4.8 | 462 | 5.8 |
| Comp. Ex. 6 | ET-6 | 5.0 | 460 | 4.9 |

As shown in Table 2, it was appreciated that the blue organic EL elements of Examples 11 to 33 using the compound according to the present invention as an auxiliary electron transport layer material exhibit significantly excellent performance in terms of current efficiency, and driving voltage compared to the blue organic EL elements of Comparative Examples 2 to 6, which include a compound with a different number of carbons contained in the dibenzo moiety from the present invention as an auxiliary electron transport layer material.

## Claims

1. A compound represented by the following Chemical Formula 1: 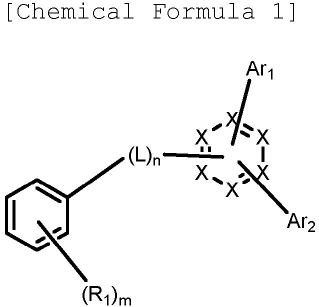 wherein in Chemical Formula 1,
a plurality of X are the same as or different from each other, and are each independently CR₂ or N, provided that at least two of the plurality of X are N,
R₂ is selected from the group consisting of hydrogen, deuterium (D), halogen, a cyano group, a nitro group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ monoarylphosphinyl group, a C₆ to C₆₀ diarylphosphinyl group, a C₆ to C₆₀ arylamine group, a C₅ to C₆₀ arylheteroarylamine group, and a heteroarylamine group having 5 to 60 nuclear atoms,
L is a single bond, or is selected from the group consisting of a C₆-C₁₈ arylene group and a heteroarylene group having 5 to 18 nuclear atoms,
R₁ is selected from the group consisting of a C₆ to C₆₀ aryl group, and a heteroaryl group having 5 to 60 nuclear atoms,
m is an integer of 1 or more, n is an integer of 0 to 3, Ar₂ is selected from the group consisting of a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ monoarylphosphinyl group, a C₆ to C₆₀ diarylphosphinyl group, a C₆ to C₆₀ arylamine group, a C₅ to C₆₀ arylheteroarylamine group, and a heteroarylamine group having 5 to 60 nuclear atoms,
Ar₁ is substituent represented by the following Chemical Formula 2, 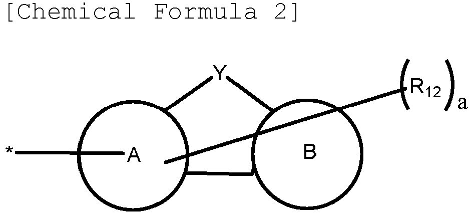 in Chemical Formula 2,
* indicates a site where a bond with Chemical Formula 1 is made,
Y is O, S or NR₁₁,
Ring A and Ring B are the same as or different from each other, each independently being a C₅ to C₁₈ monocyclic or polycyclic hydrocarbon ring that may or may not include heteroatoms,
R₁₁ and R₁₂ are the same as or different from each other, each independently being selected from: a C₁ to C₁₂ alkyl group, a C₆ to C₁₂ aryl group, and a heteroaryl group having 5 to 12 nuclear atoms, or combine with an adjacent group to form a fused ring,
a is an integer of 0 to 2,
the arylene and heteroarylene groups of L; the aryl, heteroaryl groups of R₁; the alkyl, alkenyl, alkynyl, cycloalkyl, heterocycloalkyl, aryl, heteroaryl, alkyloxy, aryloxy, alkylsilyl, arylsilyl, alkylboron, arylboron, arylphosphine, monoarylphosphinyl, diarylphosphinyl, arylamine, arylheteroarylamine, and heteroarylamine groups of R₂, R₁₁ to R₁₂ and Ar₂ are optionally each independently unsubstituted or substituted with at least one selected from the group consisting of a deuterium (D), a halogen, a cyano group, a nitro group, a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 nuclear atoms, a C₆-C₆₀ aryl group, a heteroaryl group having 5 to 60 nuclear atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a C₁-C₄₀ arylphosphine group, a C₆-C₆₀ aryl phosphine oxide group, a C₆-C₆₀ arylamine group, a C₅-C₆₀ 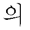 arylheteroarylamine group, and a heteroarylamine group having 5 to 60 nuclear atoms, and provided that when the substituent is present in a plural number, they are optionally the same or different from each other, and
the total number of carbon atoms contained in Ar₁ substituted or unsubstituted with the substituents comprises 16 to 24.

2. The compound of claim 1, wherein Ar₁ is selected from substituents represented by the following structural formulas: 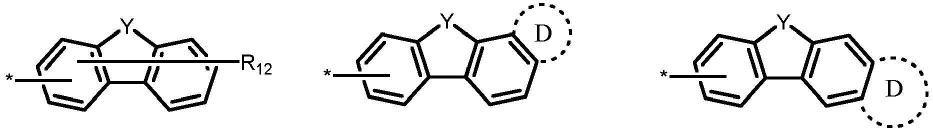 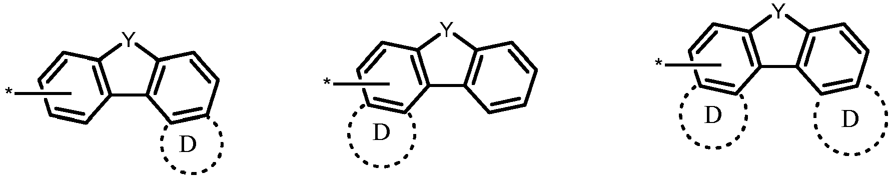 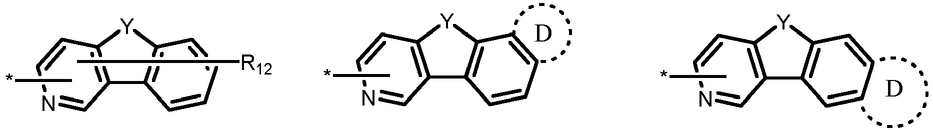 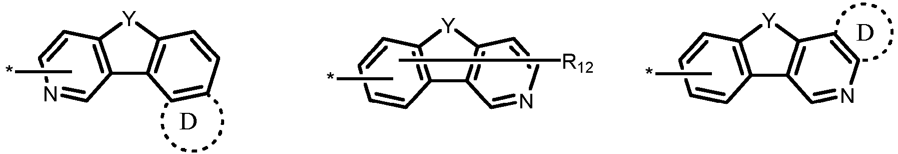 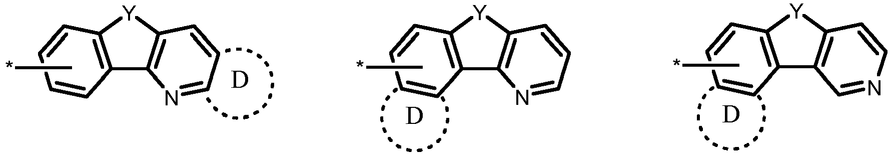 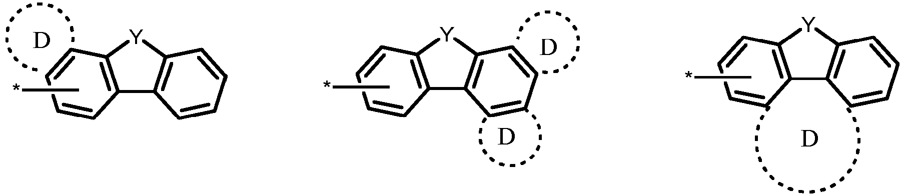 wherein in the above structural formulas,
* indicates a site where a bond with Chemical Formula 1 is made,
Ring D are the same or different from each other, each independently being a monocyclic or polycyclic hydrocarbon ring that may or may not include heteroatom, and
Y and R₁₂ are each as defined in claim 1.

3. The compound of claim 1, wherein Ar₁ is selected from substituents represented by the following structural formulas: 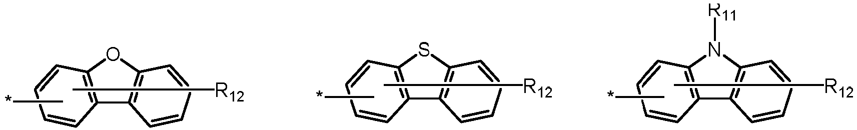 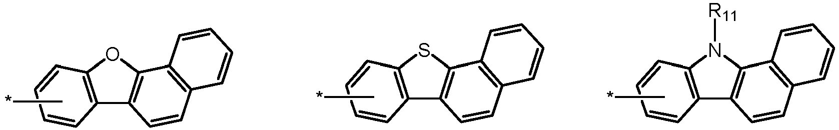 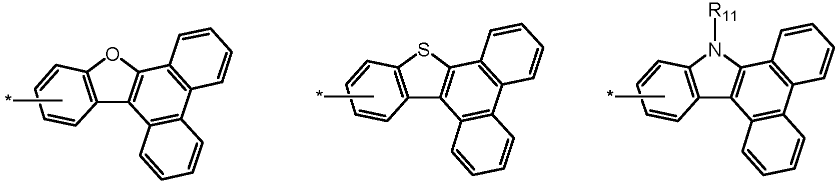 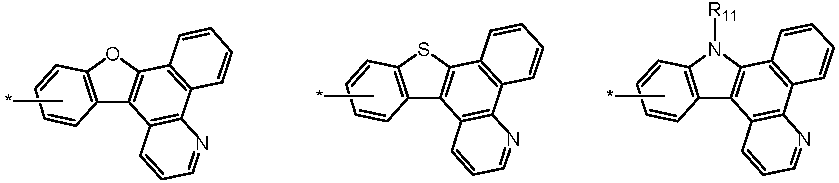 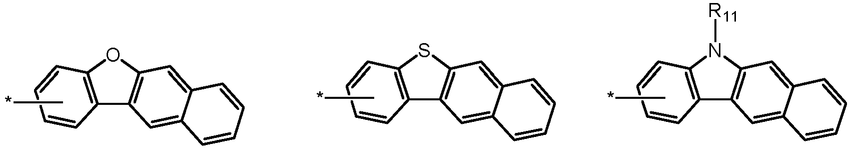 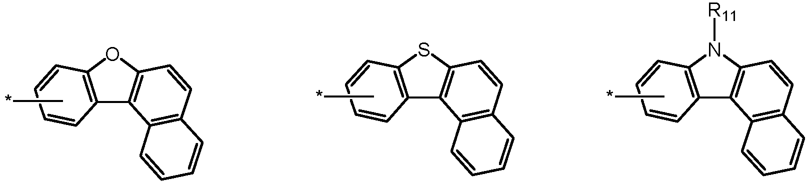 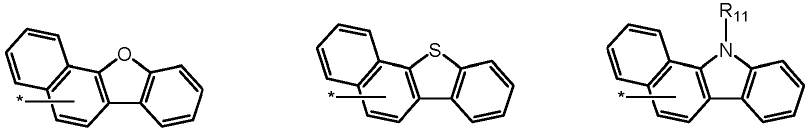 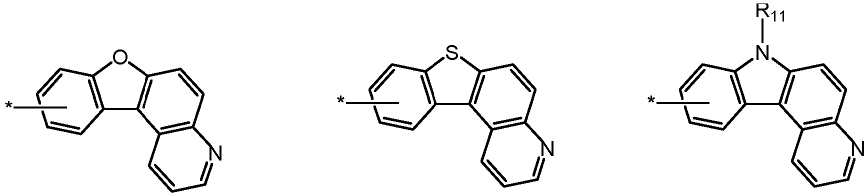 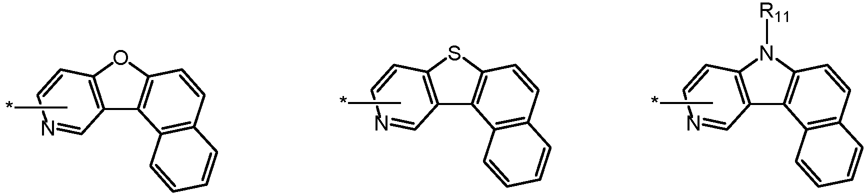 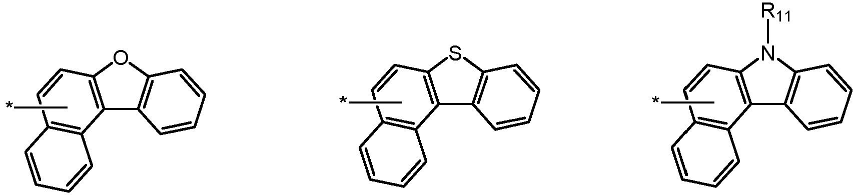 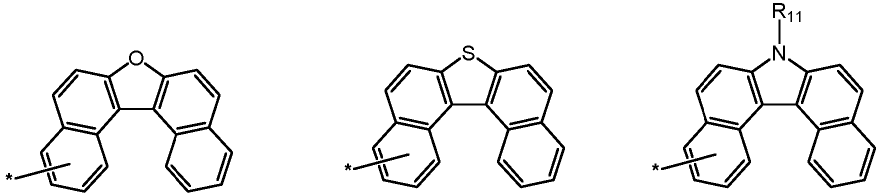 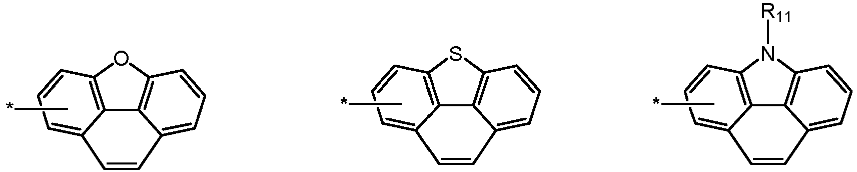 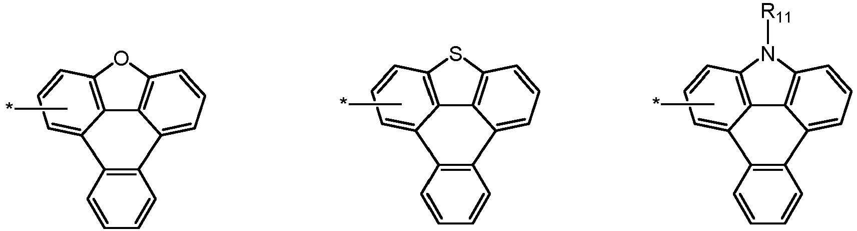 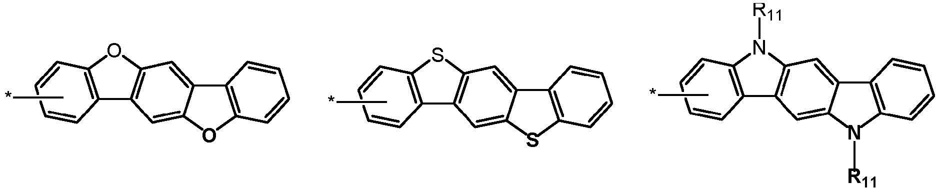 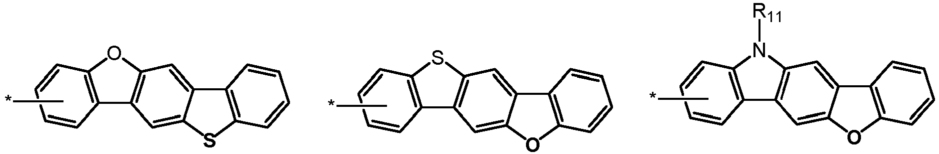 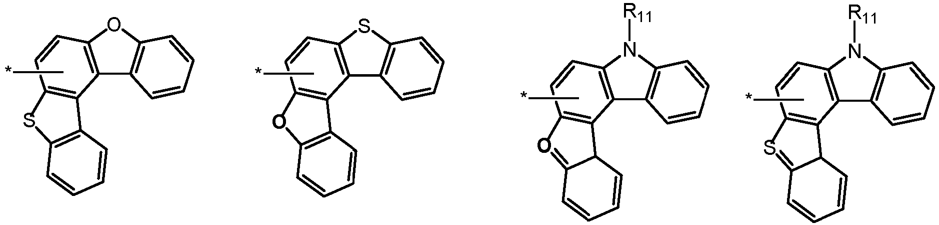 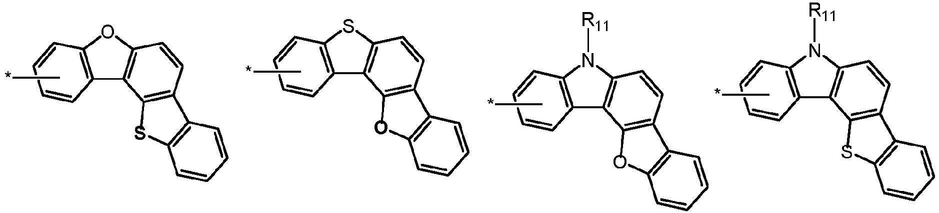 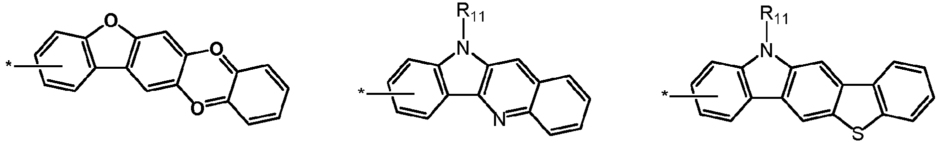 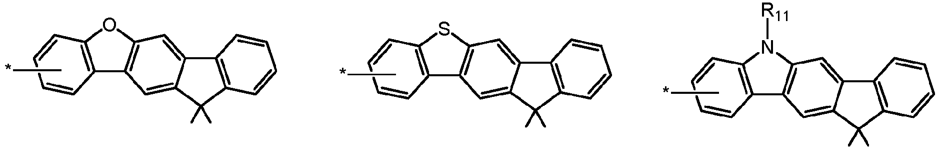 wherein in the above structural formulas,
* indicates a site where a bond with Chemical Formula 1 is made, and
R₁₁ and R₁₂ are each as defined in claim 1.

4. The compound of claim 1, wherein R₁ is a C₆ to C₁₂ aryl group,
m is an integer from 1 to 5, and
the total number of carbon atoms contained in R₁ substituted or unsubstituted with the substituents comprises at least 18.

5. The compound of claim 1, wherein X containing ring is selected from substituents represented by the following structural formulas: 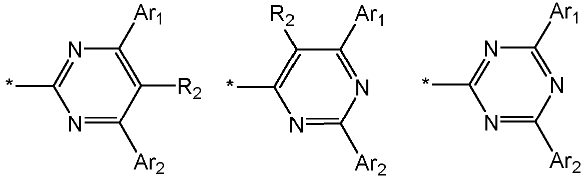 wherein in the above structural formulas,
* indicates a site where a bond with Chemical Formula 1 is made, and
R₂, Ar₁ and Ar₂ are each as defined in claim 1.

6. The compound of claim 1, wherein Ar₂ is selected from the group consisting of a C₆ to C₆₀ aryl group and a heteroaryl group having 5 to 60 nuclear atoms.

7. The compound of claim 1, wherein Ar₂ is selected from substituents represented by the following structural formulas:

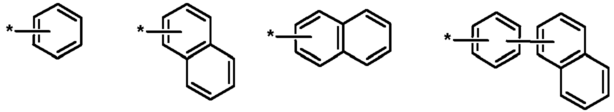

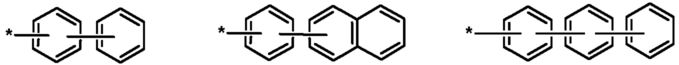

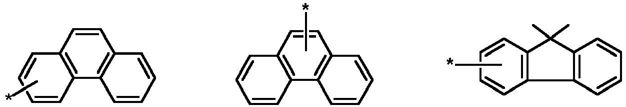

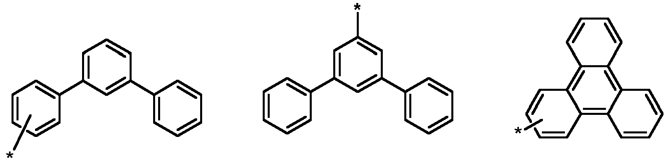

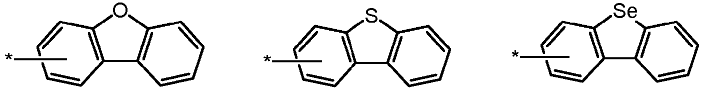

8. The compound of claim 1, wherein the compound represented by Chemical Formula 1 is represented by any one of Chemical Formulas 3 to 9 below: 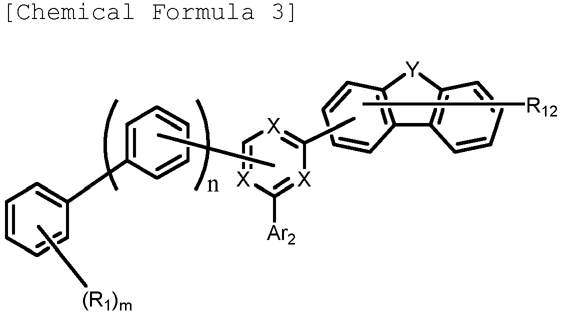 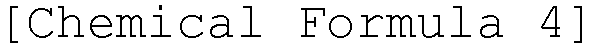 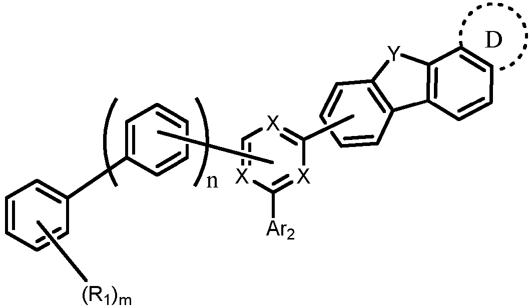 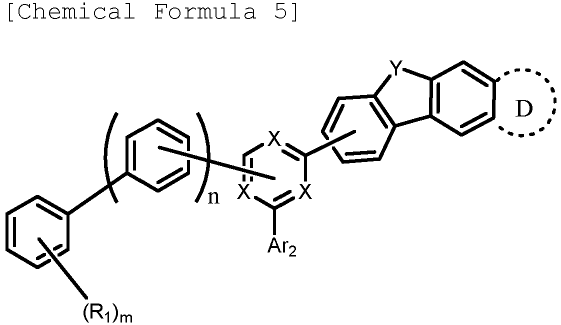 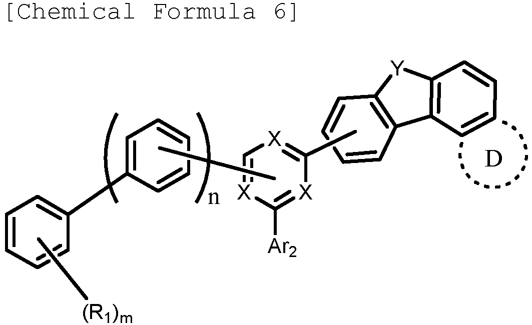 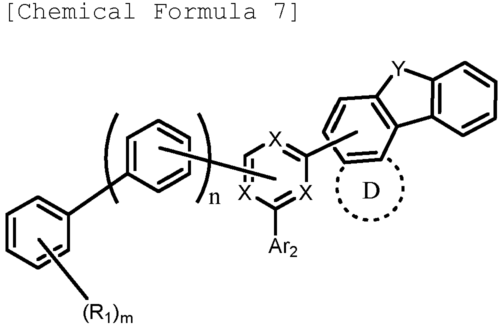 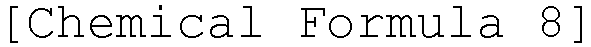 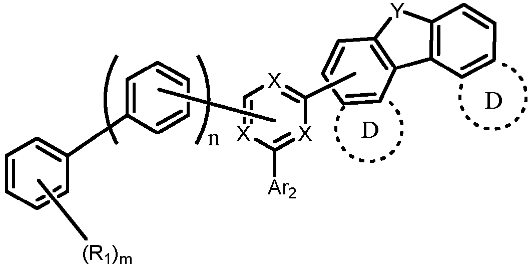 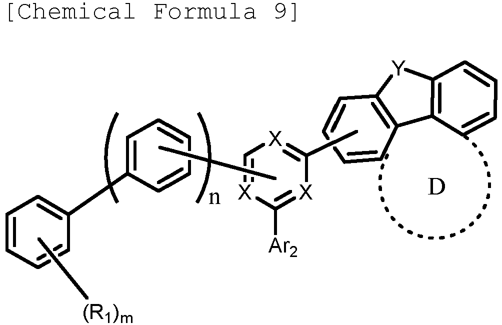 wherein in Chemical Formulas 3 to 9,
X, Y, Ar₂, R₁, n and m are each as defined in claim 1, and
Ring D is monocyclic or polycyclic hydrocarbon ring that may or may not include heteroatom.

9. The compound of claim 1, wherein the compound represented by Chemical Formula 1 is represented by any one of Chemical Formulas 10 to 21 below: 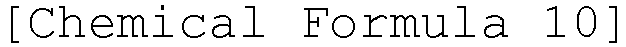 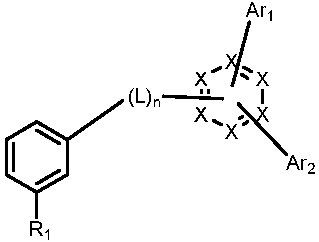 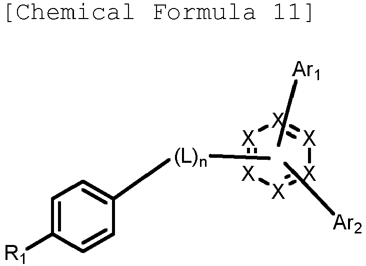 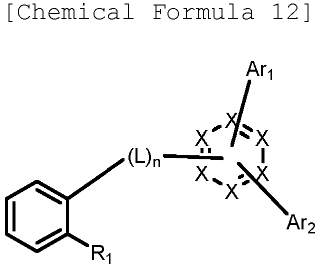 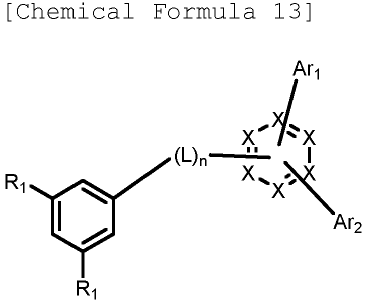 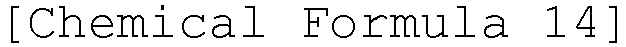 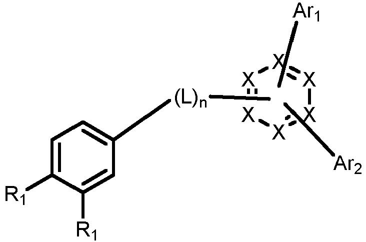 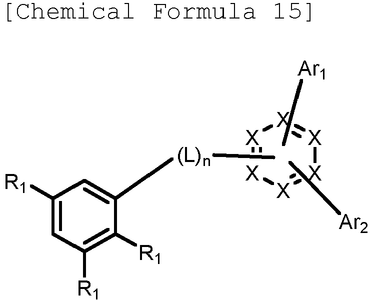 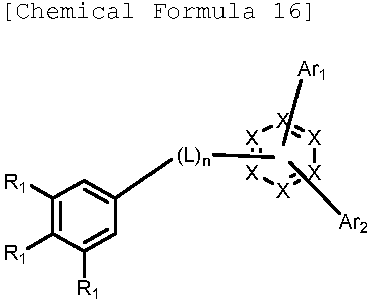 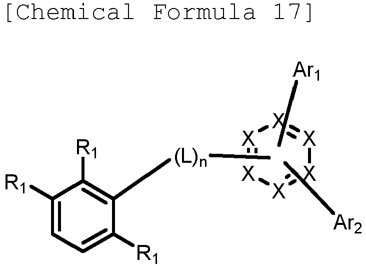 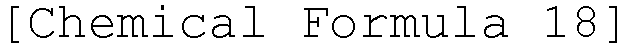 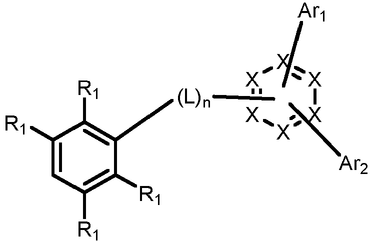 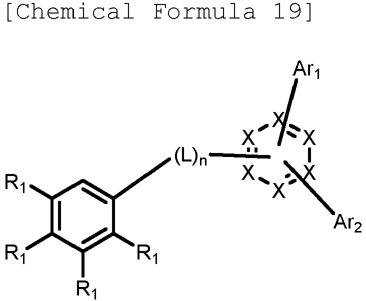 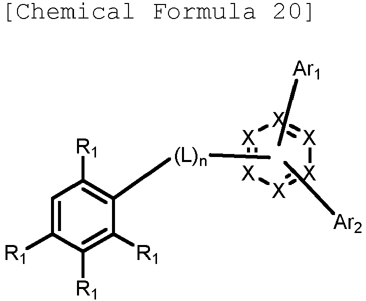 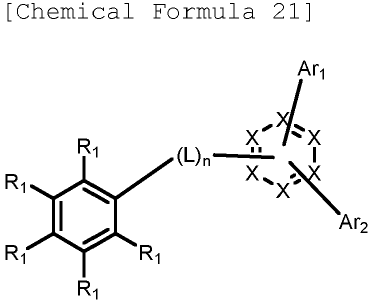 wherein in Chemical Formulas 10 to 21,
X, Ar₂, Ar₂, L, R₁, and n are each as defined in claim 1.

10. The compound of claim 1, wherein the compound represented by Chemical Formula 1 is selected from compounds represented by any one of the following Chemical Formulas 1 to 93.

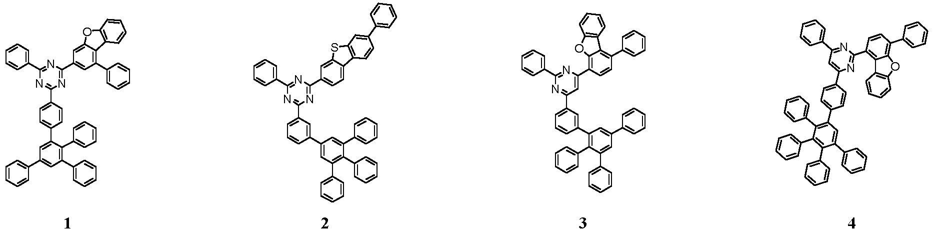

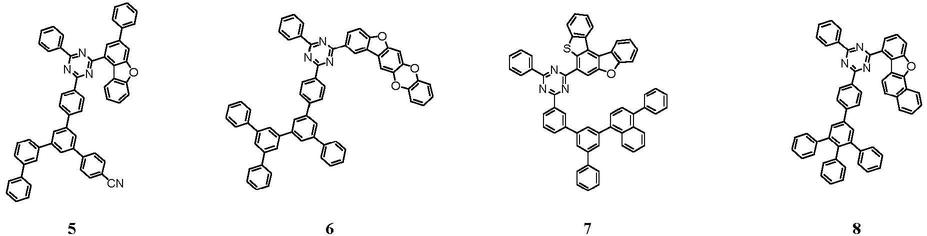

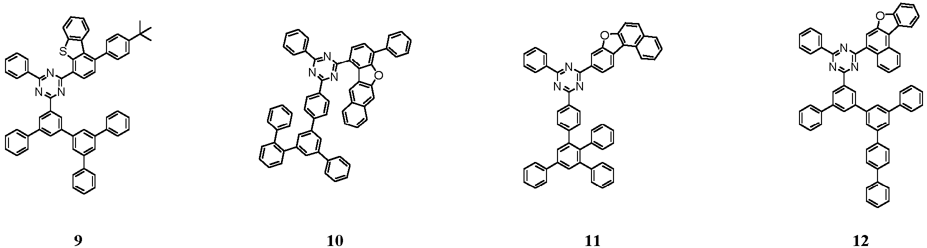

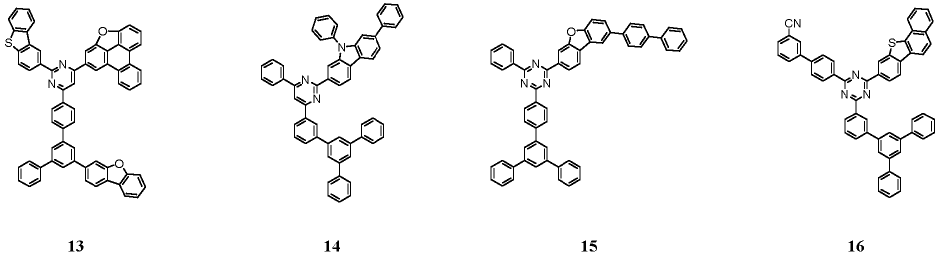

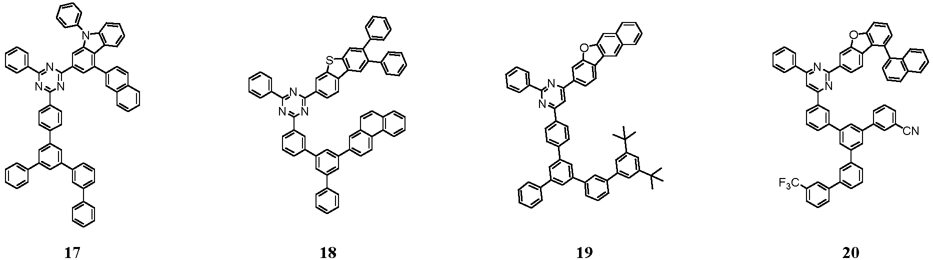

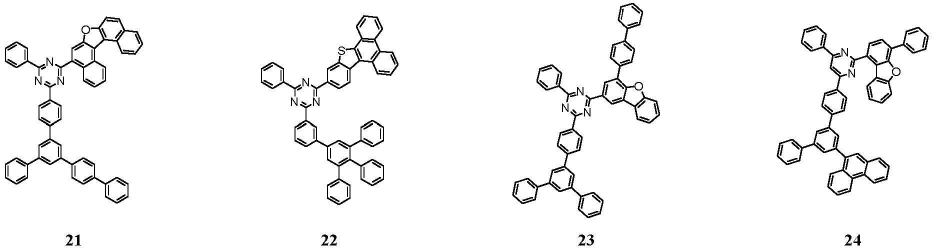

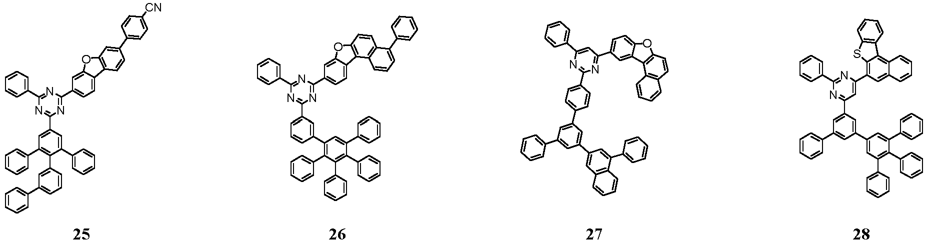

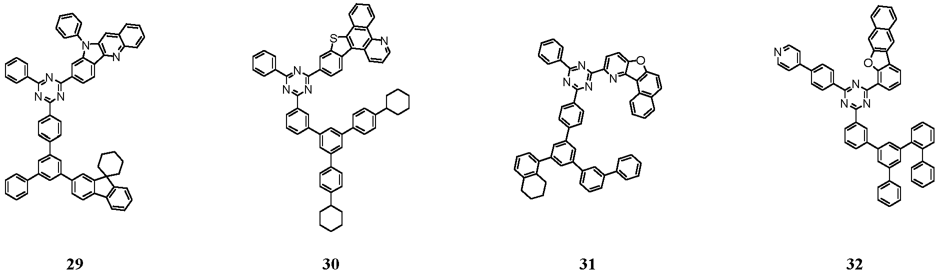

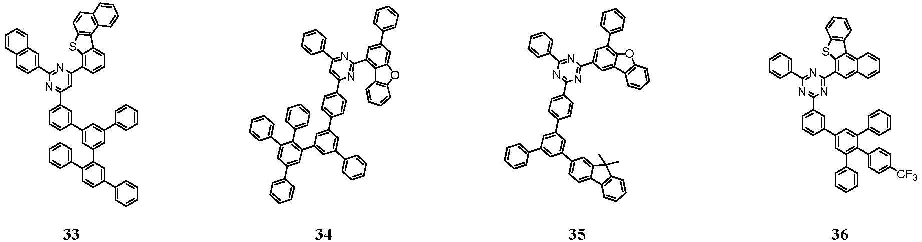

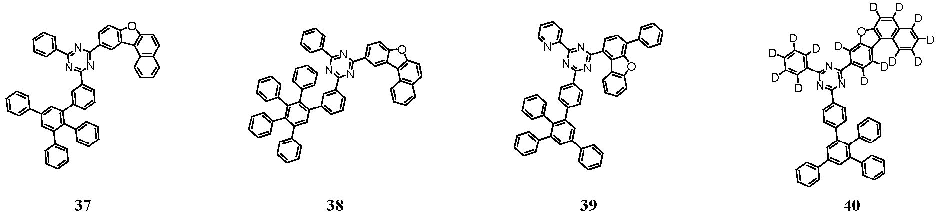

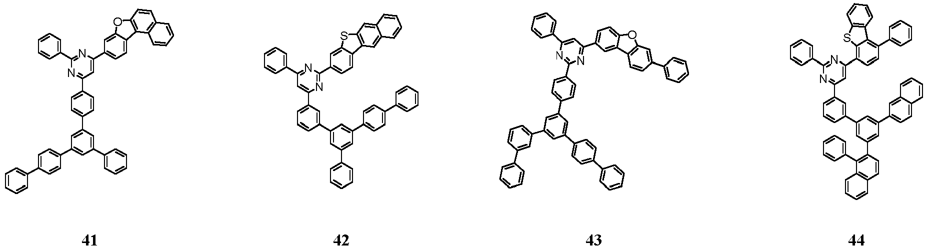

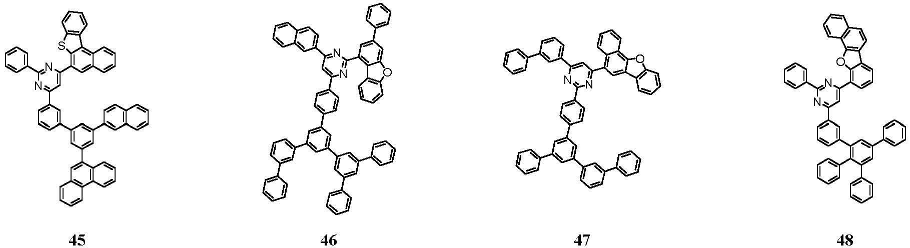

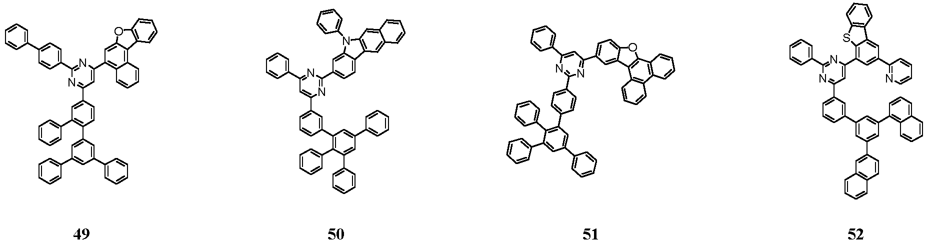

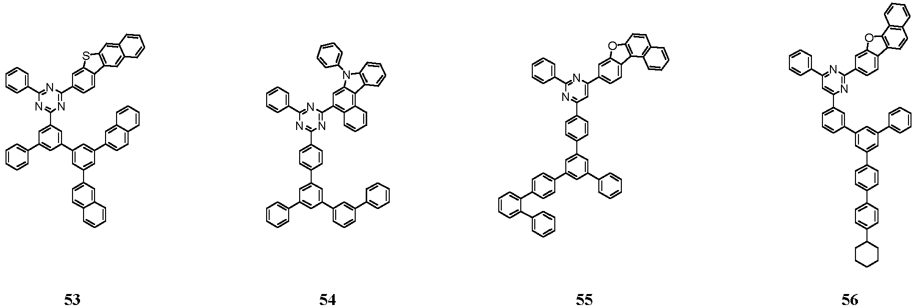

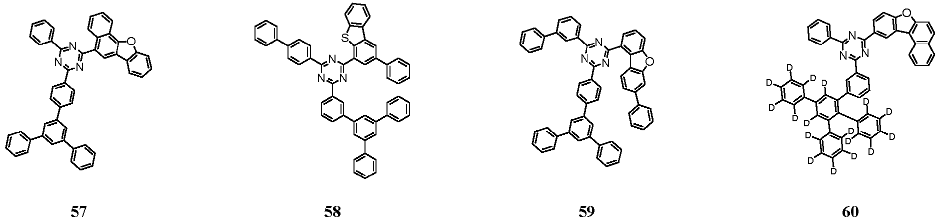

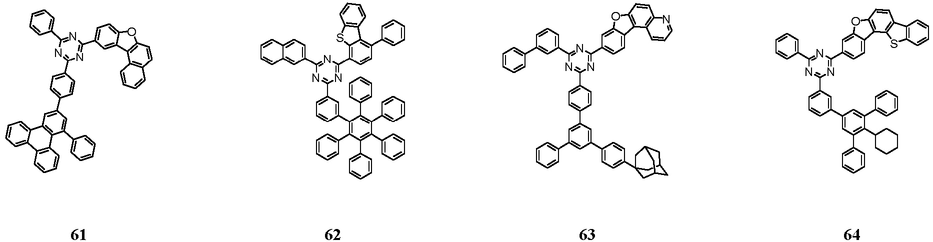

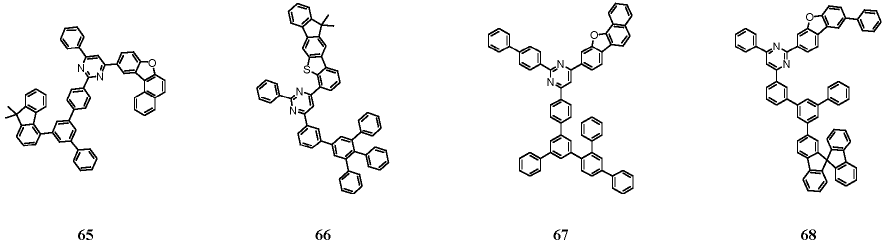

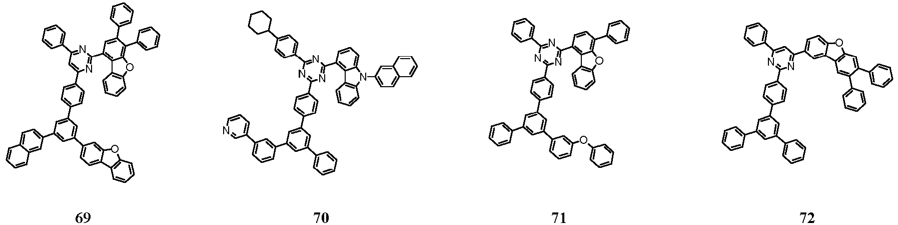

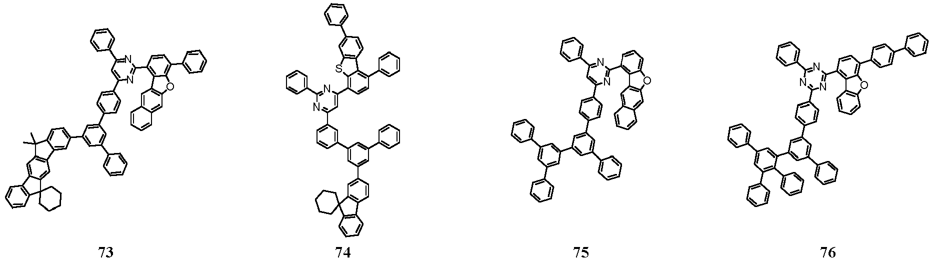

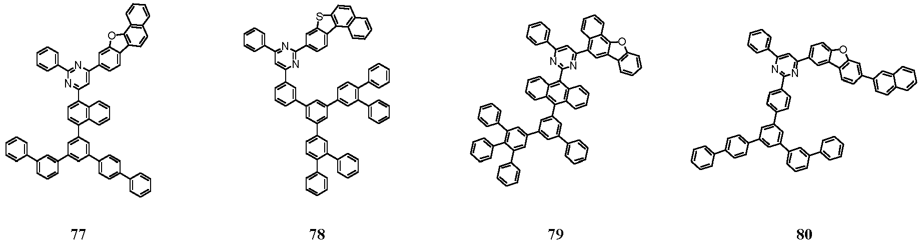

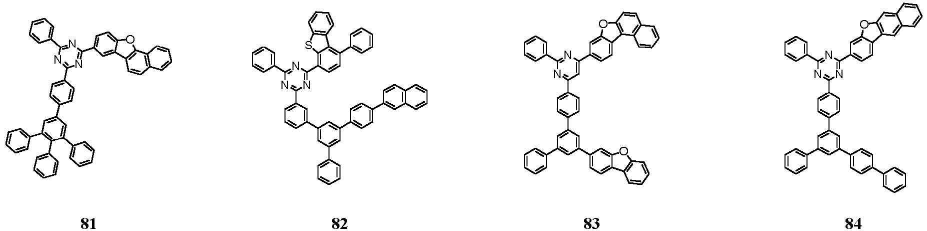

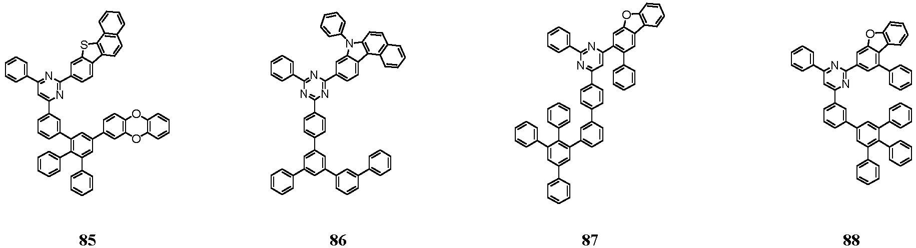

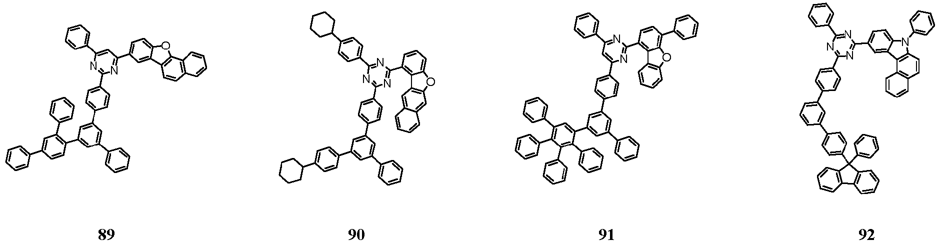

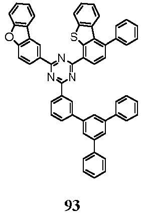

11. The compound of claim 1, wherein the compound represented by Chemical Formula 1 is a light emitting layer material, an electron transport layer material or an auxiliary electron transport layer material.

12. An organic electroluminescent element comprising: an anode, a cathode, and one or more organic layers disposed between the anode and the cathode, wherein at least one of the one or more organic layers comprises the compound according to any one of claims 1 to 11.

13. The organic electroluminescent element of claim 12, wherein the organic layer comprising the compound is selected from: a light emitting layer, a light emitting auxiliary layer, a hole injection layer, a hole transport layer, an electron injection layer, a lifespan enhancement layer, an electron transport layer, and an auxiliary electron transport layer.

14. The organic electroluminescent element of claim 13, wherein the compound is included as at least one material of a phosphorescent host material of a light emitting layer, an electron transport material and an auxiliary electron transport layer.
